# EUROPÄISCHE PATENTSCHRIFT

(11) **EP 3 273 802 B1**
(45) Veröffentlichungstag und Bekanntmachung des Hinweises auf die Patenterteilung: **25.06.2025**
(21) Anmeldenummer: 15722432.0
(22) Anmeldetag: 27.03.2015
(51) Int. Cl.: A23L 5/20, C12N 9/02

(54) **VERWENDUNG EINER TRICHOTHECENE TRANSFORMIERENDEN ALKOHOLDEHYDROGENASE, VERFAHREN ZUR TRANSFORMATION VON TRICHOTHECENEN SOWIE TRICHOTHECENE TRANSFORMIERENDER ZUSATZSTOFF**
USE OF A TRICHOTHECENE-TRANSFORMING ALCOHOL DEHYDROGENASE, METHOD FOR TRANSFORMING TRICHOTHECENES AND TRICHOTHECENE-TRANSFORMING ADDITIVE
UTILISATION D'UN ALCOOL DÉSHYDROGÉNASE TRANSFORMANT LES TRICHOTHÉCÈNES, PROCÉDÉ DE TRANSFORMATION DE TRICHOTHÉCÈNES ET ADDITIF TRANSFORMANT LES TRICHOTHÉCÈNES

(43) Veröffentlichungstag der Anmeldung: 31.01.2018
(73) Patentinhaber: DSM Austria GmbH, 3131 Getzersdorf bei Traismauer (AT)
(72) Erfinder: BINDER, Eva-Maria, 3430 Tulln (AT); WEBER, Barbara, 1150 Wien (AT); BERNARD, Claudia, 3484 Grafenwörth (AT)
(74) Vertreter: Cunow, Gerda
(86) Internationale Anmeldenummer: PCT/AT2015/000048
(87) Internationale Veröffentlichungsnummer: WO 2016/154640

(56) Entgegenhaltungen:
- WO-A1-2009/133461
- WO-A1-2014/180939
- US-A1- 2006 105 061
- DATABASE WPI Week 200377, Derwent World Patents Index; AN 2003-818681, XP002744030
- MUKHERJEE PRANAB K ET AL: "Alcohol dehydrogenase restricts the ability of the pathogen Candida albicans to form a biofilm on catheter surfaces through an ethanol-based mechanism", INFECTION AND IMMUNITY, AMERICAN SOCIETY FOR MICROBIOLOGY, US, vol. 74, no. 7, 1 July 2006 (2006-07-01), pages 3804 - 3816, XP002543233, ISSN: 0019-9567, DOI: 10.1128/IAI.00161-06

## Beschreibung

Die vorliegende Erfindung bezieht sich auf die Verwendung einer Trichothecene transformierenden Alkoholdehydrogenase, ein Verfahren zur Transformation von Trichothecenen sowie einen Trichothecene transformierenden Zusatzstoff.

Trichothecene stellen eine häufig vorkommende Gruppe von Mykotoxinen dar, zu der unter anderem Deoxynivalenol (DON, CAS Nr. 51481-10-8), T-2 Toxin (CAS Nr. 21259-20-1), HT-2 Toxin (CAS Nr. 26934-87-2), Nivalenol (CAS Nr. 23282-20-4), Fuseranon X (CAS Nr. 23255-69-8), Scripentriol, 15-Acetoxyscirpenol (CAS Nr. 2623-22-5), 4,15-Diacetoxyscirpenol (CAS Nr. 2270-40-8), Trichodermol (CAS Nr. 2198-93-8), Verrucarin A (CAS Nr. 3148-09-2), Verrucarin J (CAS Nr. 4643-58-7), Isotrichodermin (CAS Nr. 91423-90-4), Hydroxyisotrichodermin (CAS Nr. 344781-02-8), Calonectrin (CAS Nr. 38818-51-8), T-2 Tetraol (CAS Nr. 34114-99-3), Deacetylneosolaniol (CAS Nr. 74833-39-9), Neosolaniol (CAS Nr. 36519-25-2), Acetylneosolaniol (CAS Nr. 65041-92-1), Sporotrichiol (CAS Nr. 101401-89-2), Trichotriol (CAS Nr. 109890-37-1), Sambucinol (CAS Nr. 90044-33-0) und Culmorin (CAS Nr. 18374-83-9) zählen. Trichothecene, insbesondere DON, auch bekannt als Vomitoxin, können durch eine Vielzahl von Fusarien-Pilzen, insbesondere *F. graminearum* und *F. culmorum,* produziert werden. Diese Pilze befallen beispielsweise Kulturpflanzen, wie Mais, diverse Getreidearten, wie Weizen, Hafer oder Gerste, wobei in der Regel der Pilzbefall vor der Ernte auftritt und das Pilzwachstum bzw. die Mykotoxinbildung vor bzw. bei nicht sachgemäßer Lagerung auch nach der Ernte erfolgen kann.

Die Food and Agriculture Organization (FAO) schätzt, dass weltweit 25 % der agrarischen Produkte mit Mykotoxinen kontaminiert sind, was zu erheblichen wirtschaftlichen Einbußen führt. In einer aktuelleren weltweit durchgeführten Studie von I. Rodrigues und K. Naehrer, Toxins, 2012, 4, 663-675 wurden in dem Zeitraum von Jänner 2009 bis Dezember 2011 insgesamt 23.781 Proben analysiert, wobei 81 % positiv auf mindestens ein Mykotoxin und 59 % positiv auf Trichothecene, insbesondere DON getestet wurden. Trichothecene, insbesondere DON konnte in allen Regionen der Welt ebenso wie in allen getesteten Getreide- und Futtermittelklassen, wie beispielsweise Mais, Sojamehl, Weizen, Weizenkleie, DDGS (Trockenschlempe) sowie in Fertigfuttermischungen mit einer Häufigkeit von bis zu 100 % gefunden werden. Abgesehen von nicht prozessierten Grundnahrungsmitteln wurden Trichothecene auch in verarbeiteten Lebensmitteln wie Mehl, Frühstücksflocken, Teigwaren, Brot, Feingebäck, getreidebasierter Kinder- und Babynahrung nachgewiesen.

Trichothecene besitzen folgende Strukturformel, 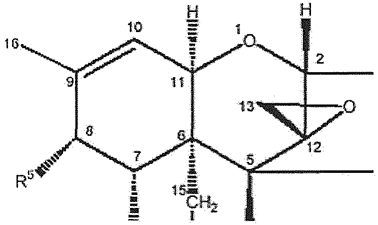 wobei die unterschiedlichen Substitutionsreste R1 bis R5 je nach Trichothecentyp verschieden sind. Es ist bekannt, dass neben der Epoxygruppe auch eine intakte α-Hydroxygruppe am C-3 Atom der Trichothecene für deren toxische Wirkung mitverantwortlich ist. Trichothecentypen mit einer Hydroxygruppe am C-3 Atom sind unter anderem Deoxynivalenol, T-2 Toxin, HT-2 Toxin, Nivalenol, Fuseranon X, 15-Acetoxyscirpenol, 4,15-Diacetoxyscirpenol, Trichodermol, T-2 Tetraol, Deacetylneosolaniol, Acetylneosolaniol, Sporotrichiol, Trichotriol, Sambucinol und Culmorin.

Deoynivalenol (DON) besitzt eine charakteristische Carbonylgruppe am C-8 Atom und hat folgende Strukturformel 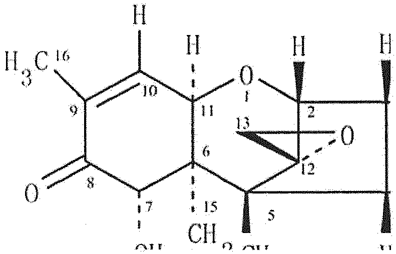 sowie die IUPAC Bezeichnung (3α,7α)-3,7,15-trihydroxy-12,13-epoxytrichothec-9-en-8-one. In der Natur kommen auch mehrere toxische DON-Subtypen mit einer Hydroxygruppe am C-3 Atom vor. Diese sind z.B. acetyliertes DON (z.B. 15-acyl-DON), glycosyliertes DON, sulfoniertes DON (z.B. DONS-1, DONS-2) oder DON-Sulfate (DON-15-Sulfat). Auch diese DON-Subtypen werden zu den Trichothecentypen mit einer Hydroxygruppe oder substituierten Hydroxygruppe am C-3 Atom gezählt.

Aufgrund der toxischen Wirkung von DON sind Grenz- bzw. Richtwerte für Lebensmittel und Futtermittel von den zuständigen Behörden definiert worden. So hat die Europäische Union den DON-Gehalt in Lebensmittel reglementiert (EC No. 1881/2006, EC No. 1126/2007) und für Futtermittel Richtwerte (2006/576/EC) empfohlen. In den USA hat die FDA empfohlene maximale Richtwerte veröffentlicht.

Krankheiten, die durch Aufnahme von Mykotoxinen bei Menschen oder Tieren verursacht werden, werden als Mykotoxikosen bezeichnet. Im Fall von Trichothecenen oder Trichothecentypen werden diese auch als Trichothecen-Mykotoxikosen, spezifischer als von durch am C-3 Atom eine Hydroxygruppe aufweisenden Trichothecenen versursachten Mykotoxikosen oder noch spezifischer als DON-Mykotoxikosen bezeichnet. Es ist bekannt, dass die toxischen Wirkungen von Trichothecenen auf Tiere und Menschen auf mehreren Faktoren beruhen. Darunter sind die Inhibierung der Proteinbiosynthese, die möglichen Interaktionen mit Serotonin- und Dopaminrezeptoren, sowie die Hochregulierung von pro-inflammatorischen Zytokinen (EFSA Journal 2004, 73, 1-41). DON-Mykotoxikosen verursachen des Weiteren Veränderungen von Biomarkern, wie dem Anstieg der IgA Konzentration in Blut, dem Anstieg der SOCS3 Konzentration in der Leber oder der Reduktion von IGFALS Levels im Plasma (Pestka et al. 2004, Toxicol. Lett. 153, 61-73) sowie der Reduktion der Claudinkonzentration im Darm (Pinton et al. 2009, Tox. Appl. Pharmacol. 237, 41-48) diagnostiziert werden.

Trichothecen-Mykotoxikosen zeigen sich z.B. bei Schweinen in einer reduzierten Futteraufnahme, einer Reduktion des Wachstums, einem Auftreten von Erbrechen und Durchfall sowie in einer gestörten Immunfunktion und Nährstoffabsorption im Darm. Bei Geflügel bewirken Trichothecen-Mykotoxikosen unter anderem eine Verschlechterung der Futteraufnahme und der Gewichtszunahme, das Auftreten von Durchfall, sowie eine Reduktion des Eierschalengewichts. Bei Wiederkäuern wurden die Reduktion der Futteraufnahme und die Verringerung der Milchproduktion beschrieben. In Aquakultur bewirken Trichothecen-Mykotoxikosen bei Fischen (z.B. Lachs, Wels oder Forelle) und Shrimps unter anderem eine Verschlechterung der Futteraufnahme und der Wachstumsraten (Binder et. al, Guide to Mykotoxins; ISBN 978-0-9573721-0-8). Toxische Effekte bei Hunden und Katzen sind ebenfalls beschrieben (EFSA Journal 2004, 73, 1-41). Beim Menschen können Trichothecen-Mykotoxikosen unter anderem Übelkeit, Erbrechen, Durchfall, abdominale Schmerzen, Kopfweh oder Fieber verursachen (Sobrova et. al, Interdisc. Toxicol. 2010, 3 (3), 94-99).

Die primäre Strategie zur Reduktion einer Trichothecen- bzw. DON-Kontamination von Nahrungs- oder Futtermitteln ist die Einschränkung des Pilzbefalls, beispielsweise durch Einhaltung der "guten landwirtschaftlichen Praxis". Dazu zählt unter anderem, der Einsatz von Saatgut, das frei von Schädlingen und Pilzbefall ist oder das Einpflügen von Ernterückständen. Des Weiteren kann durch den korrekten Einsatz von Fungiziden das Pilzwachstum auf dem Feld reduziert werden. Nach der Ernte sollte das Erntegut bei einer Restfeuchtigkeit von unter 15 % und geringer Temperatur gelagert werden, um das Pilzwachstum zu verhindern. Ebenso sollte vor der Weiterverarbeitung durch Pilzbefall kontaminiertes Gut entfernt werden. Trotz dieser Liste von Maßnahmen berichteten I. Rodriges und K. Naehrer (2012), dass selbst in Regionen mit den höchsten landwirtschaftlichen Standards, wie den USA und Zentraleuropa in den Jahren 2009 bis 2011 79 % bzw. 72 % aller getesteten Maisproben mit DON kontaminiert waren. bis 2011 79 % bzw. 72 % aller getesteten Maisproben mit DON kontaminiert waren. Aus dem Dokument MUKHERJEE PRANAB K ET AL: "Alcohol dehydroenase restricts the ability of the pathogen Canadida albicans to form a biofilm on catheter surfaces through an ethanolbased mechanism", INFECTION AND IMMUNITY, AMERICAN SOCIETY FOR MCROBIOLOGY, US, Bd. 74, Nr. 7, 1. Juli 2006 (2006-07-01) ist bekannt geworden, das eine Alkoholdehydrogenase in der Lage ist, die Fähigkeit von Canadida albicans zur Ausbildung von Biofilmen zu verstärken.

In der US 2006/105061 A1 wird eine galenische Zusammensetzung zur Verhinderung einer metallinduzierten Leberschädigung, einer neoplastischen Lebererkrankung und einer Tumorinduktion beschrieben, umfassend *Panax pseudoginseng*, *Eucommiae ulmoides* und *Polygonati rhizoma.*

In der WO 2009/133461 A1 wird eine Antifouling-Zusammensetzung zur Hemmung der Bildung eines Biofilms, umfassend ein Enzym beschrieben.

In der WO 2014/180939 A1 sind ein Verfahren und Mittel zum Aufrechterhalten und Konservieren der enzymatischen Aktivität einer Dehydrogenase beschrieben.

Weitere Möglichkeiten zur Reduktion der Mykotoxinbelastung in Nahrungs- oder Futtermitteln sind deren Adsorption bzw. Transformation. Für die Adsorption ist es erforderlich, dass die Bindung des Mykotoxins an das Adsorbens stark und spezifisch über einen weiten pH-Bereich ist und während des gesamten Verdauungsprozesses im gastrointestinalen Bereich stabil bleibt. Obwohl einige nicht-biologische Adsorptionsmittel, wie z.B. Aktivkohle, Silikate oder synthetische Polymere, wie Cholestyramin, für Aflatoxine effizient eingesetzt werden können, ist ihr Einsatz für andere Mykotoxine, insbesondere für Trichothecene, nicht effektiv. Biologische Adsorptionsmittel, wie z.B. Hefe oder Hefeextrakte sind in der Literatur ebenfalls beschrieben, haben jedoch eine ähnliche Limitierung wie nicht-biologische Adsorptionsmittel. Ein wesentlicher Nachteil von Adsorptionsmitteln ist deren mögliche nicht-spezifische Bindung von anderen Molekülen, die essentiell für die Ernährung sein können.

Auch die Transformation, insbesondere die Detoxifikation von Trichothecenen durch physikalische und chemische Behandlungen ist limitiert, da DON sehr stabil ist und selbst bei Temperaturbehandlungen von bis zu 350 °C stabil bleibt.

Eine mögliche mikrobielle Transformation von DON wurde in der EP-B 1 042 449 beschrieben, gemäß welcher der Mikroorganismus BBSH 797 (DSM 11798) zur Entgiftung von DON eingesetzt wird. Die Detoxifizierung beruht hierbei auf der Öffnung des Epoxidrings am C-12 und C-13 Atom von DON. Die US 2012/0263827 A beschreibt die Biotransformation von DON zu 3-epi-DON durch einen Mikroorganismus mit der internationalen kanadischen Hinterlegungsnummer 040408-1. Bei vielen futter- oder lebensmitteltechnologischen Prozessen ist jedoch eine Beimengung von Mikroorganismen oder Adsorptionsmittel nicht möglich oder vom Gesetzgeber nicht gestattet, so dass dort eine Transformation bzw. eine Detoxifikation von Trichothecenen, wie von DON oder DON-Subtypen nicht möglich ist.

Trichothecene, wie DON und DON-Subtypen werden rasch im Magen-Darm-Trakt vom menschlichen oder tierischen Körper aufgenommen, weshalb eine schnelle und zielgerichtete Detoxifizierung wichtig ist.

Die Alkoholdehydrogenase der SEQ ID-Nr. 1 wurde erstmals in der JP-A 2003/159079 zur Herstellung von 2-Keto-Gulonsäure beschrieben. Die WO 2009/133464 beschreibt ein Verfahren zur Oxidation von Sacchariden mittels dem Enzym der SEQ ID-Nr. 1 in Nahrungs- oder Futtermittel zur Oxidation von Stärke, insbesondere in der Backwarenindustrie zur Verlangsamung des Alterungsprozesses von Brot. Die Alkoholdehydrogenase wird hierbei für die Oxidation von Hydroxygruppen von Kohlenhydraten eingesetzt.

Die Alkoholdehydrogenasen mit den SEQ ID-Nr. 2 und 3 wurde im Zuge einer Genomsequenzierungen von Mikroorganismen *Devosia sp.* identifiziert und sind online am Server des National Center for Biotechnology Information (NCBI) unter der Identifikationsnummern GI:737041022 und GI:630002266 hinterlegt. Eine genauere Charakterisierung der Alkoholdehydrogenasen mit den SEQ ID-Nrn. 2 und 3 erfolgte im Zuge dieser Arbeiten nicht.

Aufgrund der Vielzahl von toxischen Wirkungen von Trichothecenen sowie der Häufigkeit ihres Vorkommens besteht somit ein Bedarf an Stoffen bzw. Stoffgruppen, wie Enzymen, die zur spezifischen, sicheren und zuverlässigen Transformation, insbesondere Detoxifizierung von Trichothecenen herangezogen werden können.

Die vorliegende Erfindung zielt auf die Verwendung einer spezifischen Alkoholdehydrogenase sowie Varianten davon ab, mit denen es gelingt, wenigstens ein am C-3 Atom eine Hydroxygruppe aufweisendes Trichothecen in weniger toxische Produkte zu transformieren.

Zur Lösung der Aufgabe hat sich überraschenderweise gezeigt, dass die Verwendung von einer Metallionen und einen Chinoncofaktor enthaltenden Alkoholdehydrogenase der SEQ ID-Nr. 1 oder dazu eine Sequenzidentität von wenigstens 80 %, bevorzugt wenigstens 86 %, insbesondere bevorzugt wenigstens 89 % aufweisenden, funktionellen Variante und wenigstens einem Redoxcofaktor zur Transformation von wenigstens einem am C-3 Atom eine Hydroxygruppe aufweisenden Trichothecen es ermöglicht, am C-3 Atom eine Hydroxygruppe aufweisende Trichothecene, wie DON, T-2 Toxin oder Nivalenol spezifisch und zuverlässig zu transformieren.

Unter einer Transformation wird verstanden, wenn die Struktur von Toxinen verändert wird, wodurch die Toxine bevorzugt in nicht bzw. weniger toxische Metaboliten umgewandelt, d.h. transformiert werden. Im vorliegenden Fall erfolgt die Strukturänderung insbesondere am C-3 Atom, der am C-3 Atom eine Hydroxygruppe aufweisenden Trichothecene, durch die katalytische Umwandlung der C-3 Hydroxygruppe in eine Ketogruppe. Überraschenderweise gelingt durch die erfindungsgemäße Verwendung der Alkoholdehydrogenase eine Transformation von am C-3 Atom eine Hydroxygruppe aufweisende Trichothecenen, insbesondere von DON, in unterschiedlichsten chemischen und biologischen Milieus, wie z.B. in Puffer, Futtermittelbrei, Speichel, Futtermittel enthaltendem Magensaft bzw. in Futtermittel enthaltendem Darminhalt. Dies ist außergewöhnlich, da in den jeweiligen Milieus für die enzymatische Aktivität wichtige Parameter wie der pH-Wert, die Proteasekonzentration, Ionenstärke oder Substanz-Matrices äußerst unterschiedlich sind. Dadurch kann eine Aktivität des Enzyms vom Versetzen des Nahrungs- oder Futtermittels mit Wasser, über dessen orale Aufnahme und auch im Mund-Magen-Darm-Trakt gewährleistet werden. Überraschend ist, dass für bestimmte Milieus auf eine externe Zugabe von Redoxcofaktoren verzichtet werden kann, dies gilt insbesondere für Futtermischungen, Speichel sowie Magensaft Die Alkoholdehydrogenase der SEQ ID-Nr.1 ist eine Chinoncofaktor-abhängige Alkoholdehydrogenase. Zur Herstellung eines aktives Holoenzyms bzw. einer aktiven Alkoholdehydrogenase muss ein Chinoncofaktor, bevorzugt Pyrrolochinolinchinon (PCC) in Anwesenheit eines Metallions, vorzugsweise Ca2+, an das Enzym gebunden werden.Die aktivierte Alkoholdehydrogenase enthält daher sowohl den Chinoncofaktor als auch das Metallion, wobei das molare Verhältnis von Enzym zu Chinoncofaktor bei 1:1 liegt.Des weiteren wird für die katalytische Aktivität des Alkoholdehydrogenase ein Redoxcofaktor benötigt, wobei dieser entweder in Form eines synthetisch hergestellten Redoxcofaktors zusätzlich zu der aktivierten Alkoholdehydrogenase eingesetzt wird, oder ein bereits im Nahrungs- oder Futtermitteln sowie in Sekreten von Tieren oder Menschen vorliegender Redoxcofaktor herangezogen werden kann. Diese natürlichen Redoxcofaktoren können z.B. aus Nahrungs- oder Futtermitteln im Zuge der Nahrungs- oder Futtermittelzuberei-tung, -verarbeitung oder -verdauung im Mund-Magen-Darmtrakt von Menschen oder Tieren gebildet und ggf. daraus extrahiert werden. Menschliche oder tierische Sekrete, die einen derartigen natürlichen Redoxcofaktor enthalten sind z.B. Verdauungssekrete wie Speichel, Magensaft, Darmsaft, Bauchspeichel, Gallenflüssigkeit oder Pansensaft.

Die Ausdrücke "Polypeptidvariante" oder "Variante" beziehen sich auf funktionelle Polypeptide, die im Vergleich zur SEQ ID-Nr. 1 wenigstens eine Aminosäuresubstitution besitzen, wobei die enzymatische Funktion beibehalten bleibt. Als enzymatische Funktion wird die Transformation, insbesondere die Oxidation der Hydroxygruppe am C-3 Atom von Trichothecenen zu einer Ketogruppe verstanden. Des Weiteren kann eine "Polypeptidvariante" zusätzlich Aminosäureinsertionen oder -deletionen, insbesondere eine C- oder N-terminal verlängerte oder verkürzte Sequenz relativ zur Polypeptidsequenz der SEQ ID-Nr. 1, besitzen. Eine enzymatische Funktion ist dann "im Wesentlichen beibehalten", wenn der enzymatische Reaktionsmechanismus unverändert bleibt, d.h. das Trichothecen an derselben Stelle oxidiert wird und die enzymatische Aktivität der Variante wenigstens 10 %, bevorzugt wenigstens 50 %, bevorzugter wenigstens 90 %, insbesondere >100 % bezogen auf das ursprüngliche, parentale Polypeptid der SEQ ID-Nr. 1 beträgt.

Die Bezeichnung "Sequenzidentität" bezieht sich auf eine prozentuale Sequenzidentität. Für Aminosäuresequenzen und Nukleotidsequenzen kann die Sequenzidentität visuell bestimmt, bevorzugt aber mit einem Computerprogramm errechnet werden. Als Referenzsequenz wird die Aminosäuresequenz der SEQ ID-Nr. 1 definiert. Der Sequenzvergleich wird auch innerhalb von Sequenzabschnitten durchgeführt, wobei als Abschnitt eine durchgängige Sequenz der Referenzsequenz zu verstehen ist. Die Länge der Sequenzabschnitte beträgt für Peptidsequenzen normalerweise 3 bis 200, bevorzugter 15 bis 65, am bevorzugtesten 30 bis 50 Aminosäuren. Es gibt eine Vielzahl von käuflichen oder kostenfrei verfügbaren bioinformatischen Programmen, die zur Homologiebestimmung herangezogen werden können und kontinuierlich weiterentwickelt werden. Beispiele hierfür sind GCG Wisconsin Bestfit package (Devereux et al. 1984), BLAST (Altschul et al. 1990) oder BLAST 2 (Tatusova und Madden 1999). Aufgrund der unterschiedlichen Einstellmöglichkeiten dieser Algorithmen ist es möglich, dass diese bei gleichen Inputsequenzen zu unterschiedlichen Ergebnissen kommen. Daher muss der Suchalgorithmus und die dazugehörigen Einstellung definiert werden. Im vorliegenden Fall wurde die Sequenzidentität mit Hilfe des Programms NCBI BLAST (Basic Local Alignment Search Tool), insbesondere mit BLASTP für Polypeptide, welches auf der Homepage des "National Center for Biotechnology Information" (NCBI; http://www.ncbi.nlm.nih.gov/) zur Verfügung gestellt wird, durchgeführt. Damit ist es möglich zwei oder mehrere Sequenzen miteinander nach dem Algorithmus von Altschul et al., 1997 (Nucleic Acids Res., 25:3389-3402) zu vergleichen. Hierbei wurden die Programme in der Version vom 12. Aug. 2014 verwendet. Als Programmeinstellungen wurden die Basiseinstellungen herangezogen, insbesondere für den Aminosäuresequenzvergleich: "max target sequence" = 100; "expected treshold" = 10; "word size" = 3; "matrix" = BLOSOM62; "gap costs" = "Existence: 11; Extention: 1"; "computational adjustment" = "Conditional compositional score matrix adjustment".

Durch die erfindungsgemäße Verwendung der Metallionen und einen Chinoncofaktor enthaltenden Alkoholdehydrogenase oder einer funktionellen Variante davon gelingt es wenigstens 20 %, bevorzugt wenigstens 50 %, insbesondere wenigstens 90 % von wenigstens einem am C-3 Atom eine Hydroxygruppe aufweisenden Trichothecen, insbesondere DON, zu transformieren wobei es hierfür ausreichend ist die Metallionen und einen Chinoncofaktor enthaltende Alkoholdehydrogenase oder eine funktionelle Variante davon mit wenigstens einem am C-3 Atom eine Hydroxygruppe aufweisenden Trichothecen für wenigstens eine Minute, bevorzugt wenigstens 5 Minuten, insbesondere wenigstens 60 Minuten in Kontakt zu bringen.

Gemäß einer Weiterbildung der Erfindung wird die Aminosäuresequenz der funktionellen Variante gewählt aus der Gruppe der SEQ ID-Nrn. 2 bis 4 verwendet. Mit diesen funktionellen Varianten, die zur Alkoholdehydrogenase der SEQ ID-Nr. 1 eine Sequenzidentität von wenigstens 86 % besitzen, gelingt es am C-3 Atom eine Hydroxygruppe aufweisende Trichothecene, insbesondere DON, mit gleichbleibend guten Ergebnissen zu transformieren.

Entsprechend einer Weiterbildung der Erfindung, wird der Chinoncofaktor gewählt aus der Gruppe PCC, TTC, TPC, LTC und CTC, vorzugsweise PCC verwendet. Indem einer der Chinoncofaktoren Pyrrolochinolinchinon (PCC, CAS Nr. 72909-34-3), Tryptophan Tryptophylchinon (TTQ, CAS Nr. 134645-25-3), Topachinon (TPC, CAS Nr. 64192-68-3), Lysintyrosylchinon (LTQ, CAS Nr. 178989-72-5) oder Cysteintryptophylchinon (CTC, CAS Nr. 400616-72-0) in den Alkoholdehydrogenasen verwendet wird, gelingt es am C-3 Atom eine Hydroxygruppe aufweisende Trichothecene, wie DON, zu nicht-toxischen bzw. vom toxikologischen Standpunkt unbedenklichen Derivaten zu transformieren.

Eine besonders rasche und vollständige Bindung des Chinoncofaktors an die Alkoholdehydrogenase wird dadurch erreicht, dass er mittels wenigstens eines Metallions gewählt aus der Gruppe Li⁺, Na⁺, K⁺, Mg²⁺, Ca²⁺, Zn²⁺, Zn³⁺, Mn²⁺, Mn³⁺, Fe²⁺,Fe³⁺, Cu²⁺, Cu³⁺, Co²⁺ und Co³, vorzugsweise Ca²⁺ und Mg²⁺ gebunden wird.

Indem, wie dies einer Weiterbildung der Erfindung entspricht, zusätzlich wenigstens ein Redoxcofaktor gewählt aus der Gruppe Phenazinmethosulfat (PMS), PMS-Derivate, Kaliumhexacyanidoferrat (III), Natriumhexacyanidoferrat (III), Cytochrome C, Coenzym Q1, Coenzym Q10, Methylenblau und N,N,N',N'-Tetramethyl-p-phenylenediamin (TMPD), vorzugsweise Phenazinmethosulfat (PMS, CAS Nr.: 299-11-6), Coenzym Q1 und Coenzym Q10, verwendet wird, gelingt eine vollständige und rasche Transformation der Trichothecene ausschließlich in Anwesenheit von Feuchtigkeit, so dass beispielsweise sichergestellt werden kann, dass in Futterbestandteilen enthaltene Trichothecene bereits während der Herstellung von Futtermitteln und jedenfalls vor deren Einsatz bei Tieren, in nicht-toxische Derivate transformiert werden. PMS-Derivate sind beispielsweise 1-Hydroxyphenazine, 2-(Pentaprenyloxy)dihydrophenazin, 5,10-Dihydro-9-dimethylallylphe-nazin-1-carbonsäure, 5,10-Dihydrophenazin-1-carbonsäure, 5-Methylphenaziniummethylsulfat, 6-Acetophenazin-1-carbonsäure, Benthophoenin, Clofazimin, Dihydromethanophenazin, Esmeraldinsäure, Esmeraldin B, Izumiphenazin A - C, Janus Grün B Kation, Methanophenazin Pelagiomicin A, Phenazin, Phenazin-1,6-dicarbonsäure, Phenazin-1-carboxamid, Phenazin-1-carbonsäure, Phenosafranin, Pyocyanin, Saphenamycin, Saphensäure oder Saphensäuremethylester. Dadurch, dass die Transformation von am C-3 Atom eine Hydroxygruppe aufweisenden Trichothecenen in Nahrungs- oder Futtermitteln, insbesondere Futtermitteln für Schweine, Geflügel, Rinder, Pferde, Fische, Aquakultur und Haustiere sowie in für die zur Herstellung oder Verarbeitung von Nahrungs-oder Futtermitteln eingesetzten pflanzlichen Rohstoffen durchgeführt wird, gelingt es gesundheitliche Schäden an Tieren und Menschen durch die erfindungsgemäße Verwendung zu vermeiden.

Die vorliegende Erfindung zielt weiterhin darauf ab, ein Verfahren zur Verfügung zu stellen, mit dem es gelingt Trichothecene, insbesondere am C-3 Atom eine Hydroxygruppe aufweisende Trichothecene sicher und zuverlässig, unabhängig davon, ob die agrarischen Produkte, in denen sie vorliegen, sich in einem verarbeiteten Zustand oder nicht befinden, in weniger toxische Produkte zu transformieren.

Zur Lösung dieser Aufgabe ist das erfindungsgemäße Verfahren zur enzymatischen Transformation von Trichothecenen im Wesentlichen dadurch gekennzeichnet, dass wenigstens ein am C-3 Atom eine Hydroxygruppe aufweisendes Trichothecen mit einer Metallionen und einen Chinoncofaktor enthaltenden Alkoholdehydrogenase der SEQ ID-Nr. 1 oder mit einer dazu eine Sequenzidentität von wenigstens 80 %, bevorzugt von wenigstens 86 %, insbesondere bevorzugt von wenigstens 89 % aufweisenden funktionellen Variante, mit wenigstens einem Redoxcofaktor sowie Wasser und gegebenenfalls zusätzlich mit wenigstens einem Hilfsstoff in Kontakt gebracht wird. Durch das in Kontakt bringen eines am C-3 Atom eine Hydroxygruppe aufweisenden Trichothecens mit Metallionen und einer einen Chinoncofaktor enthaltenden Alkoholdehydrogenase der SEQ ID-Nr. 1 sowie weiterhin wenigstens einem Redoxcofaktor und Wasser gelingt es, die am C-3 Atom der Trichothecene vorliegende Hydroxygruppe in ein Keton zu oxidieren, wodurch das Trichothecen als solches detoxifiziert und in eine nicht-toxische bzw. wenig toxische Verbindung transformiert wird.

Indem weiterhin anstelle der Aminosäuresequenz der SEQ ID-Nr. 1 eine funktionelle Variante davon, die aus der Gruppe der SEQ ID-Nrn. 2 bis 4 gewählt wird, eingesetzt wird, können identische Vorteile, wie durch Einsatz der Alkoholdehydrogenase der SEQ ID-Nr. 1 erreicht werden und insbesondere rasch und zuverlässig eine Transformation der in den Nahrungs- und/oder Futtermitteln, unabhängig von deren Verarbeitungszustand, d.h. ob es sich bereits um verarbeitete agrarische Produkte handelt oder nicht, enthaltenen Trichothecenen erreicht werden.

Eine besonders rasche und vollständige Transformation eines am C-3 Atom eine Hydroxygruppe aufweisenden Trichothecens wird mit dem erfindungsgemäßen Verfahren bei einer Temperatur zwischen 5 °C und 55 °C, vorzugsweise zwischen 10 °C und 50 °C, insbesondere bevorzugt zwischen 28 °C und 45 °C erreicht. Dadurch, dass das erfindungsgemäße Verfahren in einem derartigen breiten Temperaturbereich ausgeführt werden kann, steht dem Einsatz der Alkoholdehydrogenasen der SEQ ID-Nr. 1 bzw. deren funktionellen Varianten, welche wenigstens eine Sequenzidentität von mindestens 80 % zu SEQ ID-Nr. 1 aufweisen, in den unterschiedlichsten Einsatzbereichen, wie beispielsweise in der Aquakultur oder auch in technologischen Prozessen mit erhöhter Temperatur. Derartige technologische Prozesse bei denen eine Transformation von Trichothecenen bei erhöhter Temperatur wichtig ist, sind beispielsweise Verfahren zur Verarbeitung der Futtermittel, die Produktion von Teigwaren und anderen Produkten aus Mais, wie Polenta, Popcorn, Corn Flakes, Maisbrot oder Tortillas, sowie Verflüssigungsprozesse von Stärke, Saccharifizierungsprozesse oder Fermentationsprozesse, wie z.B. der Maisch- oder Gärprozess insbesondere in der Bioethanolherstellung. Wichtig ist hierbei sicherzustellen, dass die mittels dieser Prozesse hergestellten Nahrungs- oder Futtermittel keine schädlichen Mengen an am C-3 Atom eine Hydroxygruppe aufweisenden Trichothecenen enthalten.

Gemäß einer Weiterbildung des erfindungsgemäßen Verfahrens wird dieses so geführt, dass das wenigstens eine am C-3 Atom eine Hydroxygruppe aufweisende Trichothecen für wenigstens eine Minute, bevorzugt für wenigstens 5 Minuten, insbesondere bevorzugt für wenigstens 60 Minuten mit der Metallionen und einen Chinoncofaktor enthaltenden Alkoholdehydrogenase oder wenigstens einer funktionellen Variante davon, dem Redoxcofaktor, Wasser sowie gegebenenfalls dem Hilfsstoff in Kontakt gebracht wird. Indem Kontaktzeiten zwischen 1 Minute und mehr als 60 Minuten ausreichend sind, um eine ausreichende Transformation der Trichothecene in nicht-toxische bzw. wenig toxische Derivate zu erreichen, kann das erfindungsgemäße Verfahren beispielsweise in einem Verarbeitungsverfahren der agrarischen Nahrungs- oder Futtergrundmaterialien eingesetzt werden. Andererseits kann es auch beispielsweise unmittelbar vor dem Verfüttern direkt durch den Landwirt durchgeführt werden, indem beispielsweise ein Futter mit Wasser versetzt wird und dieses vor dem Verfüttern zwischen 1 Minute bis zu etwa 1 Stunde bei einer Temperatur zwischen 5 °C und 55 °C stehen gelassen wird, wodurch eine Transformation der Trichothecene in nicht-toxische Produkte in Gang gesetzt wird.

Eine besonders rasche und vollständige Transformation gelingt, wenn, wie dies einer Weiterbildung des erfindungsgemäßen Verfahrens entspricht, der Chinoncofaktor aus der Gruppe PCC, TTC, PTC, LTC und CTC, vorzugsweise PCC gewählt wird. Mit einem derartigen Chinoncofaktor wird es ermöglicht, dass die Alkoholdehydrogenasen rasch und zuverlässig die Hydroxygruppe am C-3 Atom der Trichothecene angreifen und diese in das nicht-toxische Derivat enthaltend eine Ketogruppe überführen.

Eine weitere Vervollständigung der Reaktion und insbesondere eine Beschleunigung der Reaktion gelingt, wenn in dem erfindungsgemäßen Verfahren der Cofaktor mittels wenigstens eines Metallions gewählt aus der Gruppe Li⁺, Na⁺, K⁺, Mg²⁺, Ca²⁺, Zn²⁺, Zn³⁺, Mn²⁺, Mn³⁺, Fe²⁺,Fe³⁺, Cu²⁺, Cu³⁺ , Co²⁺ und Co³⁺, vorzugsweise Ca²⁺ und Mg²⁺ an die Alkoholdehydrogenase gebunden wird. Eine derartige Verfahrensführung bewirkt nicht nur eine starke Bindung des Chinoncofaktors an der Alkoholdehydrogenase sondern erlaubt eine rasche und zuverlässige Transformation von Trichothecenen.

Für eine weitere Verbesserung der Transformation der Trichothecene, insbesondere für eine Vervollständigung der Transformationsreaktion ist das erfindungsgemäße Verfahren so weitergeführt, dass ein aus der Gruppe PMS, PMS-Derivate, Kaliumhexacyanidoferrat (III), Natriumhexacyanidoferrat (III), Cytochrome C, Coenzym Q1, Coenzym Q10, Methylenblau und TMPD, vorzugsweise PMS, Coenzym Q1 und Coenzym Q10 gewählter Redoxcofaktor eingesetzt wird. Durch Zusatz eines derartigen Redoxcofaktors gelingt es, die Transformation der am C-3 Atom einer Hydroxygruppe aufweisenden Trichothecene beispielsweise in einem wässrigen Medium durchzuführen, wie beispielsweise in einer Futteraufschlämmung oder einem Futter, welches in Aquakultur den Tieren verabreicht wird, ohne dass Redoxcofaktoren, welche beispielsweise aus Speichel, Magen- oder Darmsaft gewonnen werden könnten, zugesetzt werden müssen bzw. vorhanden sein müssen, oder das Tier die Futteraufschlämmung oder das Futter bereits aufgenommen haben muss, wodurch eine Resorption von Trichothecenen durch die das Futter aufnehmenden Tiere vermieden werden kann.

Die Erfindung zielt schließlich darauf ab, einen Trichothecene transformierenden Zusatzstoff zur Verfügung zu stellen, mit welchem es gelingt, Trichothecene in einem Futter- oder Nahrungsmittel sicher und zuverlässig in nicht-toxische Derivate zu transformieren.

Zur Lösung dieser Aufgabe ist der erfindungsgemäße Zusatzstoff im Wesentlichen dadurch gekennzeichnet, dass er eine Metallionen und einen Chinoncofaktor enthaltende Alkoholdehydrogenase der SEQ ID-Nr. 1 oder eine dazu eine Sequenzidentität von wenigstens 80 %, bevorzugt von wenigstens 86 %, insbesondere bevorzugt von wenigstens 89 % aufweisende funktionelle Variante und gegebenenfalls zusätzlich wenigstens eine weitere Komponente gewählt aus der Gruppe bestehend aus einem synthetischen Redoxcofaktor und wenigstens einem Hilfsstoff enthält. Derartige Zusatzstoffe können zu herkömmlichen Futtermitteln in geringen Konzentrationen beigemischt werden, wie beispielsweise etwa 10 g bis 1 kg auf eine Tonne Futter und ermöglichen es in einer derartigen geringen Konzentration, am C-3 Atom eine Hydroxygruppe aufweisende Trichothecene in nicht toxische Derivate zu transformieren, so dass insgesamt die Gesundheit und das Leistungsvermögen von beispielsweise Tieren, welche mit derartigen Futtermitteln gefüttert werden, verbessert wird und somit nicht nur die Ausfallsraten verringert werden können, sondern auch beispielsweise die Futterverwertung verbessert wird.

Gleichbleibend gute Ergebnisse können mit einem Zusatzstoff gemäß der Erfindung erzielt werden, in welchem anstelle der Alkoholdehydrogenase der SEQ ID-Nr. 1 eine funktionelle Variante derselben, gewählt aus der Gruppe der SEQ ID-Nrn. 2 bis 4 enthalten ist.

Für eine im Wesentlichen vollständige Transformation der an dem C-3 Atom von Trichothecenen vorliegenden Hydroxygruppe durch den erfindungsgemäßen Zusatzstoff ist dieser dahingehend weitergebildet, dass er einen Chinoncofaktor gewählt aus der Gruppe PCC, TTC, TPC, LTC und CTC enthält, ebenso wie wenigstens ein Metallion gewählt aus der Gruppe Li⁺, Na⁺, K⁺, Mg²⁺, Ca²⁺, Zn²⁺, Zn³⁺, Mn²⁺, Mn³⁺, Fe²⁺,Fe³⁺, Cu²⁺, Cu³⁺, Co²⁺ und Co³⁺. Durch eine derartige Weiterbildung gelingt es einerseits, den Chinoncofaktor sicher und zuverlässig an die Alkoholdehydrogenase zu binden und andererseits kann mit einer Alkoholdehydrogenase, die derartige Supplemente enthält, eine vollständige Transformation von Trichothecenen, wie Deoxynivalenol erreicht werden, welche eine Hydroxygruppe am C-3 Atom des Moleküls aufweisen.

Damit eine derartige Reaktion auch ohne die Anwesenheit von beispielsweise im Speichel, Magen- oder Darmsaft oder dgl. natürlich vorkommenden Redoxcofaktoren durchgeführt werden kann, ist der erfindungsgemäße Zusatzstoff dahingehend weitergebildet, dass zusätzlich als weiterer Redoxcofaktor ein synthetischer Redoxcofaktor gewählt ist, aus der Gruppe PMS, PMS-Derivate, Kaliumhexacyanidoferrat (III), Natriumhexacyanidoferrat (III), Cytochrome C, Coenzym Q1, Coenzym Q10, Methylenblau und TMPD, vorzugsweise PMS, Coenzym Q1 und Coenzym Q10 enthalten ist.

Gemäß einer Weiterbildung der Erfindung ist der Zusatzstoff so ausgebildet, dass der Hilfsstoff gewählt ist aus Gruppe von inerte Träger, Vitamine, Mineralstoffe, phytogene Substanzen, Enzyme und weitere Komponenten zur Detoxifizierung von Mykotoxinen, wie Mykotoxin-abbauende Enzyme, insbesondere Aflatoxin-Oxidasen, Ergotamin-Hydrolasen, Ergotamin-Amidasen, Zearalenon-Esterasen, Zearalenon-Lactonasen, Zearalenon-Hydrolasen, Ochratoxin-Amidasen, Fumonisin-Aminotransferasen, Fumonisin-Carboxyltransferasen, Aminopolyol-Aminoxidasen, Deoxynivalenol-Epoxidhydrolasen, Deoxynivalenol-Dehydrogenasen, Deoxynivalenol-Oxidasen, Trichothecene-Dehydrogenasen, Trichothecene-Oxidasen; und Mykotoxin-transformierenden Mikroorganismen, wie z.B. DSM 11798; und Mykotoxin-bindende Stoffe, beispielsweise mikrobielle Zellwände oder anorganische Materialien, wie Bentonite, insbesondere Smectite. Durch den Einsatz eines derartigen Zusatzstoffes kann beispielsweise in Futter- oder Nahrungsmitteln sichergestellt werden, dass die gegebenenfalls enthaltenen Mengen an am C-3 Atom eine Hydroxygruppe aufweisende Trichothecene sowie gegebenenfalls weitere Mykotoxine wie beispielsweise Fusariumtoxine, Ergotamine, Ochratoxine mit Sicherheit soweit zu detoxifizieren, dass eine schädliche Wirkung der Mykotoxine auf den Organismus des dieses Futter- oder Nahrungsmittel aufnehmenden Subjekts ausbleibt.

Weitere Einsatzbereiche der Erfindung sind Zusatzstoffe, die neben wenigstens einer erfindungsgemäßen Alkoholdehydrogenase zusätzlich wenigstens ein Enzym enthalten, das beispielsweise am Abbau von Proteinen beteiligt ist, wie z.B. Proteasen, oder das beim Metabolismus von Stärke oder Faser oder Fett oder Glycogen beteiligt ist, wie z.B. Amylase, Cellulase oder Glucanase, sowie beispielsweise Hydrolasen, lipolytische Enzyme, Mannosidasen, Oxidasen, Oxidoreduktasen, Phytasen oder Xylanasen.

Es erübrigt sich festzuhalten, dass selbstverständlich der Zusatzstoff in verkapselter oder gecoateter Form vorliegen kann, wobei für das Verkapseln oder Coaten Standardmethoden, wie z.B. in der WO 92/12645 beschrieben, herangezogen werden können. Durch das Verkapseln bzw. Coaten gelingt es, den Zusatzstoff ohne Veränderung, insbesondere ohne Abbau und Schädigung an seinen Einsatzort zu transportieren, so dass erst nach Auflösung der Schutzhülle, beispielsweise im Verdauungstrakt von Tieren, das Polypeptid zu wirken beginnt, wodurch ein noch gezielterer, rascher und vollständiger Abbau von am C-3 Atom eine Hydroxygruppe aufweisenden Trichothecenen auch im sauren, proteasereichen und anaeroben Milieu erzielt werden kann. Des Weiteren gelingt es durch die Verkapselung oder das Coaten auch die Temperaturstabilität der Alkoholdehydrogenasen im Zusatzstoff zu erhöhen wodurch dessen Einsatz beispielsweise im Pelletierprozess von Futtermitteln verbessert wird.

Der Zusatzstoffe gemäß der Erfindung kann vielfältig eingesetzt werden, wie beispielsweise zur Herstellung eines Präparats, zur Prophylaxe und/oder Behandlung von Trichothecen-Mykotoxikosen, vorzugsweise von Mykotoxikosen, welche durch Trichothecene hervorgerufen werden, die am C-3 Atom eine Hydroxygruppe aufweisen, wie insbesondere Deoxynivalenol-Mykotoxikosen. Derartigen Mykotoxikosen haben für Mensch und Tier schwerwiegende Folgen. Durch eine derartige Verwendung des Zusatzstoffs ist es im Falle der Prophylaxe möglich den Gesundheitszustand von Menschen oder Tieren trotz einer oraler Aufnahme von Trichothecenen, insbesondere von am C-3 Atom eine Hydroxygruppe aufweisende Trichothecenen, insbesondere von Deoxynivalenol im Wesentlichen auf dem Niveau zu halten, das demjenigen ohne bzw. mit einer verringerten oralen Aufnahme der Toxine entspricht. Im Falle der Behandlung von Mykotoxikosen ist es möglich die Symptome einer solchen Erkrankung zu lindern und insbesondere die SOCS3 Konzentration in der Leber bzw. die IGFALS Levels im Plasma sowie die Claudinkonzentration im Darm zu normalisieren.

Des Weiteren ist es möglich durch eine derartige Verwendung die Leistungsfähigkeit von Nutztieren, insbesondere die Futterverwertung und Gewichtszunahme zu verbessern und die Mortalitätsrate zu senken.

Die Erfindung wird nachfolgend anhand von Ausführungsbeispielen sowie einer Zeichnung näher erläutert. In dieser zeigen:
Fig. 1 die zeitliche Transformation von Deoxynivalenol für die aktivierte Alkoholdehydrogenase der SEQ ID-Nr. 1 sowie einer Kontrolle (CTR) als Vergleich, und
Fig. 2 die Darstellung der zeitlichen Transformation von DON mit den aktivierten Alkoholdehydrogenasen der SEQ ID-Nrn. 1 bis 4 sowie einer Kontrolle (CTR) als Vergleich.

### Beispiel 1: Klonierung der Gene sowie Aufreinigung der Alkoholdehydrogenase

Die für die jeweilige Wirtszelle codonoptimierten Nukleotidsequenzen der Alkoholdehydrogenasen mit den SEQ ID-Nrn. 1 bis 4 wurden von DNA2.0 bezogen und enthielten auf Nukleinsäureebene Restriktionsschnittstellen am 5' Ende und am 3'Ende der Sequenz, sowie auf Aminosäureebene zusätzlich einen C- oder N-terminalen 6xHis-Tag. Diese Nukleotidsequenzen wurden mittels Standardmethoden in Expressionsvektoren zur Expression in *Escherichia coli* bzw. *Komagataella pastoris* integriert, und in *E. coli* bzw. K. *pastoris* transformiert, sowie in *E. coli* bzw. *K. pastoris* exprimiert (J.M. Cregg, Pichia Protocols, second Edition, ISBN-10: 1588294293, 2007; J. Sambrook et al. 2012, Molecular Cloning, A Laboratory Manual 4th Edition, Cold Spring Harbor).

Die Alkoholdehydrogenasen mit den SEQ ID-Nrn. 1-4 wurden aus löslichen Zelllysaten im Falle der Expression in *E. coli* und aus der intrazellulären Expression in *K. pastoris* bzw. aus dem Kulturüberstand im Falle der extrazellulären Expression in *K. pastoris* mittels Standardmethoden chromatographisch über Nickel-Sepharosesäulen selektiv angereichert. Die selektiv angereicherten Eluate wurden in Anwesenheit von Metallionen sowie von Chinoncofaktoren inkubiert und aktiviert, wobei aktivieren bedeutet, dass die Alkoholdehydrogenasen sowohl das Metallion als auch den Chinoncofaktor gebunden aufweisen. Für die Bestimmung der enzymatischen Eigenschaften der Alkoholdehydrogenasen mit den SEQ ID-Nrn. 1 bis 4 in den nachfolgenden Beispielen 3 bis 7 wurden diese aktivierten Alkoholdehydrogenasen herangezogen. Die Bestimmung der Gesamtproteinkonzentration erfolgte photometrisch mit dem Bradford-Reagenz (Sigma # B6916), wobei die Absorptionen in einem Mikrotiterplatten Photometer (plate reader, Biotek, Synergy HT) bei einer Wellenlänge von 595 nm gemessen wurde. Die Proteinkonzentration wurde anhand einer Kalibriergeraden ermittelt, die durch das Messen von Bovine Serum Albumin (BSA, Sigma #A4919) Lösungen mit Konzentrationen bis maximal 1500 µg/ml mit dem Bradfordassay bestimmt wurde.

### Beispiel 2: Bestimmung der Sequenzidentität

Die Bestimmung der prozentualen Sequenzidentität über die gesamte Länge der Aminosäuresequenz der Alkoholdehydrogenasen mit den SEQ ID-Nrn. 1-4 relativ zueinander wurde mit Hilfe des Programms BLAST (Basic Local Alignment Search Tool), insbesondere mit BLASTP, welches auf der Homepage des "National Center for Biotechnology Information" (NCBI; http://www.ncbi.nlm.nih.gov/) benutzt werden kann, durchgeführt, mit welchem es möglich ist zwei oder mehrere Sequenzen miteinander nach dem Algorithmus von Altschul et al., 1997 (Nucleic Acids Res. (1997) 25:3389-3402) zu vergleichen. Als Programmeinstellungen wurden die Basiseinstellungen herangezogen, insbesondere: "max target sequence" = 100; "expected treshold" = 10; "word size" = 3; "matrix" = BLOSOM62; "gap costs" = "Existence: 11; Extention: 1"; "computational adjustment" = " Conditional compositional score matrix adjustment". Die prozentualen Identitäten der Aminosäuresequenzen zueinander sind in Tabelle 1 dargestellt.

**Tabelle 1:**

| | SEQ ID-Nr. 1 | SEQ ID-Nr. 2 | SEQ ID-Nr. 3 | SEQ ID-Nr. 4 |
|---|---|---|---|---|
| SEQ ID-Nr. 1 | 100% | 87% | 89% | 86% |
| SEQ ID-Nr. 2 | 87% | 100% | 99% | 90% |
| SEQ ID-Nr. 3 | 89% | 99% | 100% | 91% |
| SEQ ID-Nr. 4 | 86% | 90% | 91% | 100% |

### Beispiel 3: Transformation von am C-3 Atom eine Hydroxygruppe aufweisenden Trichothecen

Zur Bestimmung ihrer Eignung, Trichothecene, die am C-3 Atom eine Hydroxygruppe aufweisen, insbesondere DON, Nivalenol und T-2 Toxin zu transformieren wurden die Alkoholdehydrogenasen der SEQ ID-Nrn. 1-4 mit einem C-terminalen 6xHis Tag in *E. coli,* wie in Beispiel 1 beschrieben, hergestellt.

Eine Transformation liegt dann vor, wenn die Menge von einem am C-3 Atom eine Hydroxygruppe aufweisenden Trichothecen durch das in Kontaktbringen mit einer aktivierten, d.h. Metallionen und einen Chinoncofaktor enthaltenden Alkoholdehydrogenase reduziert wird.

Jeweils 100 ml einer *E. coli* Kultur mit einer optischen Dichte (OD600 nm) von 2,0 - 2,5 wurden durch Zentrifugation bei 4 °C geerntet und in 20 ml Kaliumphosphatpuffer resuspendiert. Die Zellsuspensionen wurden durch 3-malige Behandlung mit einer French Press bei 20.000 psi lysiert. Die Zelllysate wurden durch Zentrifugieren in lösliche und unlösliche Teile getrennt. Der Überstand wurde steril filtriert und die Alkoholdehydrogenase wurde mittels Standardmethoden chromatographisch über Nickel-Sepharosesäulen selektiv angereichert. Anschließend wurde ein Pufferaustauch mittels Dialyse mit spezifischen Röhrchen mit einem Cut off von zehn Kilodalton durchgeführt. Die resultierende Gesamtproteinkonzentration wurde mittels Bradfordassay gemessen.

Das Binden der Chinoncofaktoren und der Metallionen an die Alkoholdehydrogenasen erfolgte durch Inkubation in wässriger Lösung. Der Chinoncofaktor wie beispielsweise Pyrrolochinolinchinon (PCC, CAS Nr. 72909-34-3), Tryptophan Tryptophylchinon (TTC, CAS Nr. 134645-25-3), Topachinon (TPC, CAS Nr. 64192-68-3), Lysintyrosylchinon (LTC, CAS Nr. 178989-72-5) und Cysteintryptophylchinon (CTC, CAS Nr. 400616-72-0) wird dabei als wässrige Lösung in einem etwa 20-fachen molaren Überschuss zur vorliegenden Gesamtproteinkonzentration zugegeben. Die Metallionen gewählt aus Li⁺, Na⁺, K⁺, Mg²⁺, Ca²⁺, Zn²⁺, Zn³⁺, Mn²⁺, Mn³⁺, Fe²⁺,Fe³⁺, Cu²⁺, Cu³⁺, Co²⁺ und Co³⁺ werden als wässrige Lösung eines Salzes davon eingesetzt. Wenn nicht anderes angegeben, wurden standardmäßig die Alkoholdehydrogenasen mit PCC (Sigma Aldrich #D7783) und Ca²⁺; eingesetzt als 5 mM CaCl₂-Lösung, aktiviert. Die auf diese Weise gereinigten und aktivierten Enzyme wurden für *in vitro* Transformationsansätze von einem am C-3 Atom eine Hydroxygruppe aufweisenden Trichothecen verwendet. Wenn nichts anderes angegeben ist, wird unter den Bezeichnungen "Enzym" oder "Alkoholdehydrogenase" immer die entsprechende aktivierte Metallionen und Chinoncofaktor enthaltende Alkoholdehydrogenase verstanden.

Die Transformationsansätze wurden in wässriger Lösung mit den folgenden Komponenten durchgeführt: 100 mM Tris-HCl pH 7,5 oder 10 % Teorell Stenhagen pH 7,5; synthetischer Redoxcofaktor gewählt aus der Gruppe 1 mM Phenazinmethosulfat PMS (Sigma Aldrich #P9625), 1 mM Methylenblau (Sigma #M9140), 1 mM Coenzyme Q10 (Sigma #C9538), 1 mM Coenzym Q1 (Sigma #C9538) und 20 mM Kaliumhexacyanidoferrat (III) PFC(III) (Fluka #60300); 10 ppm bis maximal 100 ppm eines am C-3 Atom eine Hydroxygruppe aufweisenden Trichothecens durch Zugabe der gewünschten Menge einer Toxin-Substratstammlösung; und 10 nM bis 100 nM, maximal 300 nM einer aktivierten Metallionen und Chinoncofaktor enthaltenden Alkoholdehydrogenase der SEQ ID-Nr. 1, 2, 3 oder 4. Wenn nicht anderes angegeben ist, wurden standardmäßig der Tris-HCl Puffer, der Redoxcofaktor PMS, DON und die Alkoholdehydrogenase der SEQ ID-Nr. 1 eingesetzt. Jeder Transformationsansatz wurde in einem 1,5 ml braunen Eppendorf-Reaktionsgefäß durchgeführt. Die Reaktionsansätze wurden bei 30 °C auf einem Thermoblock für bis zu 120 min, mindestens 40 min lang inkubiert. Zu den Zeitpunkten 0, 10, 20, 30, und 40 min wurde jeweils eine Probe von 0,1 ml entnommen und mit 0,1 ml Methanol vermischt und bei -20 °C gelagert oder alternativ unmittelbar mittels LC-MS/MS oder HPLC analysiert.

Als DON-Substratstammlösung diente eine steril filtrierte, wässrige 2000 ppm DON-Lösung. Zur Herstellung dieser Lösung wurde DON in kristalliner Form (Biopure Standard von Romer Labs, Art. Nr. 001050, Reinheit mind. 98 %) eingewogen und aufgelöst.

Zur Quantifizierung der am C-3 Atom eine Hydroxygruppe aufweisenden Trichothecene sowie deren Transformationsmetaboliten wurden HPLC Analysen durchgeführt, wobei die Substanzen mittels einer Phenomenex C18 Gemini-NX Säule mit den Dimensionen 150 mm x 4,6 mm und einer Partikelgröße von 5 µm chromatographisch getrennt wurden. Als Laufmittel diente ein Methanol-Wassergemisch mit einer Ammoniumacetat-Konzentration von 5 mM. Das UV-Signal bei 220 nm wurde aufgezeichnet und ausgewertet. Für die Quantifizierung mittels LC-MS/MS Analysen wurden die Substanzen mittels einer Zorbax eclipse C8 Säule mit den Dimensionen 150 mm x 4,6 mm und einer Partikelgröße von 5 µm chromatographisch getrennt. Als Laufmittel diente ein Methanol-Wassergemisch mit einer Ammoniumacetat-Konzentration von 5 mM. Das UV-Signal bei 220 nm wurde aufgezeichnet. Als Ionisierungsquelle diente Elektrospray Ionisation (ESI). Die am C-3 Atom eine Hydroxygruppe aufweisenden Trichothecene wurden mittels QTrap/LC/MS/MS (triple quadrupole, Applied Biosystems) im "enhanced Modus" quantifiziert.

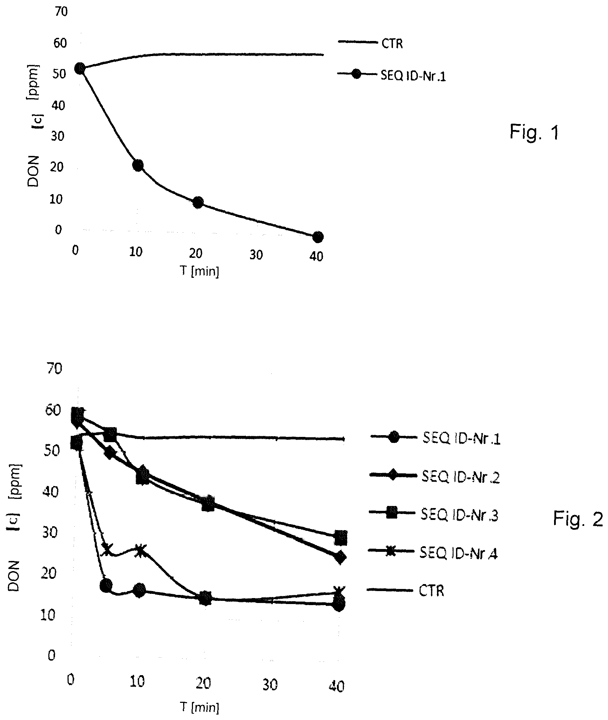

Als Maß für die Aktivität der Alkoholdehydrogenasen wurden die negativen Steigungen der Transformationsgeraden (= Abnahme der Toxinkonzentration über die Zeit) im linearen Bereich herangezogen. Zur Bestimmung der Restaktivitäten wurden die gemessen Aktivitäten bei unterschiedlichen Parametern relativ auf die Basisaktivität, gemessen unter Standardbedingungen, insbesondere 30 °C und pH 7,5, bezogen und sind in der Regel als Prozentwerte dargestellt. In Fig. 1 ist die zeitliche Transformation von DON für die aktivierte Alkoholdehydrogenase der SEQ ID-Nr. 1 und in Fig. 2 ist die zeitliche Transformation von DON für die aktivierten Alkoholdehydrogenasen der SEQ ID-Nrn. 2-4 (Fig. 1B) dargestellt. Aus den Figuren ist klar ersichtlich, dass eine Transformation von DON erfolgt, da sich die Konzentration von DON in Abhängigkeit von der Realitätszeit verringerte.

Hierbei ist in Fig. 1 die Darstellung der Transformation von DON mit der Alkoholdehydrogenase der SEQ ID-Nr. 1 in 100mM Tris HCl pH 7,5 in Anwesenheit von 50 ppm DON und 1 mM PMS gezeigt. Die Messresultate wurden durch LC-MS/MS Analysen erhalten (A) und Transformation von DON mit den Alkoholdehydrogenasen der SEQ ID-Nrn. 1-4 ist in Fig. 2 gezeigt. Die Messresultate wurden durch HPLC Analysen erhalten (B). CTR diente in den Versuchen als Negativkontrolle, welche alle Komponenten des Transformationsansatzes, bis auf die Alkoholdehydrogenasen der SEQ ID-Nr.1-4 enthielt.

Zum Vergleichen der Effizienz der Chinoncofaktoren, wurden in Transformationsansätzen jeweils 10 nM der mit den Chinoncofaktoren PCC, TTC, TPC, LTC und CTC aktivierten Alkoholdehydrogenase der SEQ ID-Nr. 1, 10 ppm DON und 1 mM synthetischer Redoxcofaktor PMS in 100 mM Tris-HCl pH 7,5 vermischt und bei 30 °C inkubiert. Die DON Konzentrationen wurden nach 30 Minuten mittels LC-MS/MS bestimmt, wobei die Resultate in Tabelle 2 dargestellt sind.

Um die Effizienz der synthetischen Redoxcofaktoren zu vergleichen, wurden in Transformationsansätzen jeweils 10 nM aktiviertes Enzym (Alkoholdehydrogenase der SEQ ID-Nr. 1), 10 ppm DON und 1 mM bzw. 20 mM der zu testenden synthetischen Redoxcofaktoren in 100 mM Tris-HCl pH 7,5 vermischt und bei 30 °C inkubiert. Die DON Konzentrationen wurden nach 30 Minuten mittels LC-MS/MS bestimmt, wobei die Resultate in Tabelle 2 dargestellt sind.

**Tabelle 2:**

| Chinoncofaktor | DON [ppm] | Redoxcofaktor | DON [ppm] |
|---|---|---|---|
| PCC | 1,94 | 1 mM PMS | 1,95 |
| TTQ | 2,32 | 20 mM PFC(III) | 2,11 |
| TPQ | 2,41 | 1 mM Coenzym Q1 | 8,58 |
| LTQ | 2,04 | 1 mM Methylenblau | 6,88 |

Um den Einfluss der Metallionen im aktivierten Enzym auf die Transformation zu testen wurde die Aktivierung der Alkoholdehydrogenase der SEQ ID-Nr. 1 und PCC, jedoch mit jeweils unterschiedlichen Metallionen, nämlich mit Mg²⁺, Ca²⁺, Zn²⁺, Mn²⁺, Fe²⁺ und Cu²⁺ durchgeführt. Die Transformationsansätze enthielten jeweils 10 nM aktivierte Alkoholdehydrogenase, 10 ppm DON und 1 mM PMS in 100 mM Tris-HCl pH 7,5 und wurden bei 30 °C inkubiert. Die DON Konzentrationen wurden nach 30 Minuten mittels LC-MS/MS bestimmt, wobei die Resultate in Tabelle 3 dargestellt sind.

**Tabelle 3:**

| Metallion | DON [ppm] | Metallion | DON [ppm] |
|---|---|---|---|
| Mg²⁺ | 1,90 | Mn²⁺ | 2,57 |
| Ca²⁺ | 1,98 | Fe²⁺ | 2,17 |
| Zn²⁺ | 2,46 | Cu²⁺ | 2,61 |

Analog zu den oben beschriebenen DON Transformationsansätzen wurden Transformationsansätze mit anderen am C-3 Atom eine Hydroxygruppe aufweisenden Trichothecenen durchgeführt. In diesen Ansätzen wurde anstatt 50 ppm DON 50 ppm T-2 Toxin bzw. 50 ppm Nivalenol eingesetzt. Alle vier Metallionen und Chinoncofaktor enthaltenden Alkoholdehydrogenasen der SEQ ID-Nr. 1 bis 4 waren in der Lage auch T-2 Toxin sowie Nivalenol zu transformieren, wobei innerhalb von 30 min jeweils mehr als die Hälfte des ursprünglich eingesetzten Toxins transformiert war.

### Beispiel 4: Messung der Aktivitätsbereiche

Zur Bestimmung der Fähigkeit der Alkoholdehydrogenasen der SEQ ID-Nrn.1-4 DON unter verschiedenen Bedingungen zu transformieren, wurde die Alkoholdehydrogenase der SEQ ID-Nr.1 exemplarisch herangezogen.

Die Alkoholdehydrogenase der SEQ ID-Nr.1 wurde wie in Beispiel 3 beschrieben hergestellt und mit Ca²⁺ und PCC aktiviert. Um die Aktivität des Enzyms über einen Temperaturbereich von 10 °C bis 50 °C und über einen pH Bereich von 3,0 bis 9,0 zu bestimmen, wurde statt des 100 mM Tris-HCl pH 7,5 Puffers ein 10%iger Teorell Stenhagen Puffer verwendet.

Die Transformationsversuche zur Bestimmung der Aktivitäten bei verschiedenen Temperaturen wurden in wässriger Lösung mit folgenden Komponenten durchgeführt: 10 % Teorell Stenhagen pH 7,5, 1 mM synthetischer Redoxcofaktor PMS, 50 ppm DON, und 10 nM aktivierter Alkoholdehydrogenase der SEQ ID-Nr. 1. Die Transformationsansätze wurden bis zu 60 min in einem Thermocycler (Eppendorf) mit einem Temperaturgradienten von 10 °C bis 50 °C inkubiert. Zu den Zeitpunkten 0, 10, 20, 30, 40 und 60 min wurde jeweils eine Probe von 0,05 ml entnommen und mit 0,05 ml Methanol gemischt, um die Reaktion zu stoppen und bei -20 °C gelagert. Die Proben wurden, so wie in Beispiel 3, beschreiben für die LC-MS/MS vorbereitet und mittels LC-MS/MS analysiert. Der Verlauf der DON-Reduktion wurde bei jeder Temperatur bestimmt und die Aktivität wurde, wie in Beispiel 3 beschrieben, berechnet. Die Steigung des linearen Bereichs der Transformationsgeraden bei 30 °C diente als Referenzwert zur Berechnung der Restaktivität bei den anderen Temperaturen. In Tabelle 4 sind die Temperaturen in °C und die zugehörigen Restaktivitäten in Prozent angegeben. Überraschenderweise hat sich gezeigt, dass die Alkoholdehydrogenase mit SEQ ID-Nr.1 über einen weiten Temperaturbereich aktiv ist. Bei 10 °C wurde eine Restaktivität von 48 % und bei etwa 50 °C von 67 % gemessen.

**Tabelle 4:**

| Temperatur [°C] | Restaktivität [%] | Temperatur [°C] | Restaktivität [%] |
|---|---|---|---|
| 10,0 | 48 | 32,8 | 105 |
| 12,7 | 60 | 33 | 108 |
| 15 | 69 | 35,3 | 105 |
| 17,6 | 73 | 38,4 | 120 |
| 20,5 | 86 | 40,7 | 116 |
| 23,3 | 89 | 43,2 | 108 |
| 26,2 | 82 | 45,9 | 96 |
| 28,3 | 100 | 48,2 | 89 |
| 30,2 | 100 | 49,8 | 67 |

Die Transformationsversuche zur Bestimmung der Aktivität in einem pH Bereich von 4,0 bis 9,0 wurden in wässriger Lösung mit folgenden Komponenten durchgeführt: 10 % Teorell Stenhagen pH 4,0 bis pH 10,0, 20 mM synthetischer Redoxcofaktor PFC(III), 100 ppm DON, und 20 nM aktivierte Alkoholdehydrogenase der SEQ ID-Nr. 1. Die Transformationsansätze wurden bis zu 60 min in einem Thermoblock bei 30 °C inkubiert. Zu den Zeitpunkten 0, 10, 20, 30, 40 und 60 min wurde jeweils eine Probe von 0,05 ml entnommen und mit 0,05 ml Methanol gemischt, um die Reaktion zu stoppen und bei -20 °C gelagert. Die Proben wurden, so wie in Beispiel 3 beschrieben, verdünnt und mittels LC-MS/MS analysiert. Der Verlauf der DON Reduktion wurde bei jedem pH Wert bestimmt und die Aktivität wurde, wie in Beispiel 3 beschrieben, berechnet. Die Steigung des linearen Bereichs der Transformationsgeraden bei pH 7,5 diente als Referenzwert zur Berechnung der Restaktivität bei den anderen pH Werten. In Tabelle 5 sind die pH Werte und die zugehörigen Restaktivitäten (DON Reduktion bezogen auf die Referenz pH Wert 7,5) in Prozent angegeben.

**Tabelle 5:**

| pH | Restaktivität | pH | Restaktivität |
|---|---|---|---|
| 4,0 | 10 % | 7,0 | 105 % |
| 5,0 | 18 % | 7,5 | 100 % |
| 6,0 | 20 % | 8,0 | 69 % |
| 6,5 | 52 % | 9,0 | 105 % |

### Beispiel 5: Bestimmung der Temperaturstabilität

Die Temperaturstabilität der Alkoholdehydrogenase der SEQ ID-Nr. 1 wurde über einen Bereich von 30 °C bis 55 °C bestimmt. Dazu wurde die aktivierte Alkoholdehydrogenase im 100 mM Tris-HCl Puffer, pH 7,5 für bis zu 60 min bei einer bestimmten Temperatur in einem Thermocycler (Eppendorf) inkubiert. Zu den Zeitpunkten 0, 5, 10, 15, 20, 30, 40 und 60 min wurde ein Aliquot der Alkoholdehydrogenase entnommen und die Aktivität in einem DON Transformationsansatz, wie in Beispiel 3 beschrieben, bestimmt. Die Transformationsansätze enthielten folgende Komponenten: 100 mM Tris-HCl, pH 7,5, 1 mM PMS, 50 ppm DON, 10 nM aktivierte Alkoholdehydrogenase der SEQ ID-Nr.1. Die Reaktionen wurden, wie in Beispiel 3 beschrieben, inkubiert und die Probenentnahme für die Aktivitätsbestimmung erfolgte nach 0; 10; 20; 30; 40 und 60 min. Der Verlauf der DON Reduktion wurde bei jeder Temperatur für jede Inkubationszeit bestimmt. Für die Bestimmung der Temperaturstabilität wurde die Steigung des linearen Bereichs der DON Transformationsgeraden berechnet. Die Steigung des linearen Bereichs der DON Transformationsgeraden der jeweiligen Temperatur zum Zeitpunkt t = 0 min wurde als Referenzwert für die Berechnung der Restaktivitäten herangezogen. In Tabelle 6 sind die Temperaturen in °C, die Inkubationszeit in Minuten und die zugehörigen Restaktivitäten in Prozent angegeben. Die Alkoholdehydrogenase der SEQ ID-Nr. 1 war bei einstündiger Lagerung bei Temperaturen von 30 °C und 37 °C am stabilsten. Im Vergleich dazu zeigte die Alkoholdehydrogenase bei 40 °C nach einer Stunde Lagerung noch 73 % Restaktivität. Nach Lagerung bei 45 °C für 30 min wurde eine 50 %ige Restaktivität gemessen. Überraschenderweise wird nach Lagerung von 5 min bei 50 °C eine Restaktivität von 84 % detektiert.

**Tabelle 6:**

| | Inkubationszeit | | | | | | | |
|---|---|---|---|---|---|---|---|---|
| | 0 min | 5 min | 10 min | 15 min | 20 min | 30 min | 40 min | 60 min |
| 30 °C | 100 % | 99 % | 98 % | 94 % | 88 % | 99 % | 100 % | 95 % |
| 37 °C | 100 % | 92 % | 94 % | 92 % | 90 % | 91 % | 47 % | 79 % |
| 40 °C | 100 % | 90 % | 83 % | 77 % | 75 % | 83 % | 82 % | 73 % |
| 45 °C | 100 % | 85 % | 78 % | 77 % | 60 % | 57 % | 47 % | 19 % |
| 50 °C | 100 % | 84 % | 30 % | 36 % | 13 % | 12 % | 10 % | 0 % |

### Beispiel 6: Bestimmung der pH-Stabilität

Die pH Stabilität der aktivierten Alkoholdehydrogenase der SEQ ID-Nr.1 wurde über einen Bereich von pH 4,0 bis pH 10,0 bestimmt. Dazu wurde die 10-fache Konzentration der aktivierten Alkoholdehydrogenase (100 nM) in 10 % Teorell Stenhagen Puffer pH 4,0 bis pH 10,0 für bis zu 120 Minuten bei einer Temperatur von 30 °C gelagert. Zu den Zeitpunkten 0, 60 und 120 min wurde ein Aliquot der Alkoholdehydrogenase entnommen und die Aktivität in einem Transformationsansatz bestimmt und wie in Beispiel 3 beschrieben, mit folgenden Komponenten bei 30 °C durchgeführt: 100 mM Tris-HCl pH 7,5, 1 mM PMS, 50 ppm DON, 10 nM aktivierte Alkoholdehydrogenase der SEQ ID-Nr.1. Die Probenentnahme für die Aktivitätsbestimmung erfolgte nach 0, 10, 20, 30 und 40 min. Der Verlauf der DON Reduktion wurde bei jedem pH Wert für jeden Zeitpunkt bestimmt. Für die Bestimmung der Stabilität wurde die Steigung des linearen Bereichs der DON Transformationsgeraden beim jeweiligen pH Wert zum jeweiligen Zeitpunkt berechnet. Die Steigung des linearen Bereichs der DON Transformationsgeraden des jeweiligen pH Werts zum Zeitpunkt t=0 min wurde als Referenzwert für die Berechnung der Aktivitäten der folgenden Inkubationszeiten herangezogen. In Tabelle 7 sind die pH Werte, die Zeit der pH-Inkubation in Minuten und die zugehörigen Restaktivitäten in Prozent dargestellt. Die Alkoholdehydrogenase der SEQ ID-Nr. 1 war nach einer 60 minütigen Inkubation bei pH 5,0 bis pH 9,0 stabil. Überraschenderweise wies die Alkoholdehydrogenase insbesondere eine gute Stabilität im sauren Milieu (kein Aktivitätsverlust bei pH 5,0) und im stark basischen Milieu (kaum Aktivitätsverlust bei nach einer 120 min Inkubation bei pH 9,0) auf.

**Tabelle 7:**

| | Inkubationszeit | |
|---|---|---|
| | 60 min | 120 min |
| pH 4,0 | 72 % | 51 % |
| pH 5,0 | 111 % | 109 % |
| pH 6,0 | 92 % | 88 % |
| pH 7,0 | 87 % | 85 % |
| pH 8,0 | 83 % | 73 % |
| pH 9,0 | 93 % | 60 % |
| pH 10,0 | 69 % | 55 % |

### Beispiel 7: Transformation von DON in komplexen Matrices

Zur Bestimmung der Fähigkeit der aktivierten Alkoholdehydrogenasen Trichothecene in komplexen Matrices auch ohne eine externe Zugabe von synthetischen Redoxcofaktoren zu transformieren, wurde die aktivierte Alkoholdehydrogenase der SEQ ID-Nr. 1, wie in Beispiel 3 beschrieben, hergestellt und DON Transformationsversuche in komplexen Matrices durchgeführt. Unter komplexen Matrices werden hier unter anderem Pansensaft von Rindern, Darminhalte aus dem Jejunum von Schweinen, Magensaft von Schweinen, Speichel von Mensch und Schwein, granuliertes Ferkelaufzuchtfutter (FAF), sowie granuliertes Ferkelaufzuchtfutter vermischt mit Speichel, Pansensaft oder Darminhalt verstanden. Um einen Vergleich zum Puffersystem zu haben, wurden Kontrollen mit Tris-HCl, wie in Beispiel 3 beschrieben, mitgeführt. Als FAF wurde ein Standardfutter auf der Basis von Mais, Soja und Gerste verwendet.

Zur Bestimmung der Alkoholdehydrogenaseaktivität in Pansensaft (pH 5,9) wurde jeweils 1 ml steriles Pansensaftfiltrat mit 100, 200 und 300 nM der aktivierten Alkoholdehydrogenase der SEQ ID-Nr. 1 und mit 50 ppm DON versetzt. Die Kontrollansätze in wässriger Lösung wurden, wie in Beispiel 3 beschrieben, ausgeführt. Alle Transformationsansätze wurden bei 30 °C auf einem Thermoblock für bis zu 24 Stunden inkubiert. Die Probennahme erfolgte zu den Zeitpunkten 0; 0,5; 1,0; 5,0 und 24,0 Stunden, wo je Zeitpunkt 0,1 ml Probe entnommen wurden und die Reaktion mit 0,1 ml Methanol gestoppt wurde. Die Proben wurden bei -20 °C gelagert, aufgetaut und für 10 min bei 13.000 Upm mit einer Eppendorf Tischzentrifuge zentrifugiert und mit einem 0,2 µM Spartan Filter steril filtriert. Für die LC-MS/MS wurden die Proben wie in Beispiel 3 verdünnt und mittels LC-MS/MS analysiert. Die Konzentration an DON zum Zeitpunkt t = 0 h, wurde als Referenzwert (100 %) für die nachfolgenden Werte herangezogen. In Tabelle 8 wird der Prozentsatz der DON-Konzentration angegeben, der zu einem bestimmten Zeitpunkt relativ zum Zeitpunkt t = 0 h gemessen wurde. Für die Aktivität im Tris-HCl Puffer ist die Anwesenheit eines extern zugegebenen synthetischen Redoxcofaktors notwendig, da die Transformation von DON langsam erfolgt und erst nach 24 Stunden mit einer Alkoholdehydrogenase-Konzentration von 300 nM detektierbar war. Überraschenderweise hat sich gezeigt, dass im sterilen Pansensaftfiltrat bei einem pH-Wert von 5,9 DON ohne die Zugabe einen externen synthetischen Redoxcofaktor transformiert wird. Dies zeigt klar, dass im Pansensaft Substanzen vorliegen, die als natürliche Redoxcofaktoren dienen. Bei einer Konzentration von 300 nM sind nach 5 stündiger Inkubation nur mehr 42 % der anfänglichen DON Menge im Ansatz enthalten. Nach 24 stündiger Inkubation ist DON bei Alkoholdehydrogenase-Konzentrationen größer als 200 nM nur in geringer Menge detektierbar.

**Tabelle 8:**

| | Pansensaft mit synthetischen Redoxcofaktor | Pansensaft ohne synthetischen Redoxcofaktor | | | Tris-HCl pH 7,5 ohne synthetischen Redoxcofaktor | | |
|---|---|---|---|---|---|---|---|
| | 100 nM | 100 nM | 200 nM | 300 nM | 100 nM | 200 nM | 300 nM |
| 0 h | 100 % | 100 % | 100 % | 100 % | 100 % | 100 % | 100 % |
| 0,5 h | 0 % | 100 % | 100 % | 87 % | 100 % | 99 % | 95 % |
| 1,0 h | 0 % | 100 % | 100 % | 83 % | 100 % | 99 % | 89 % |
| 5,0 h | 0 % | 94 % | 75 % | 42 % | 99 % | 88 % | 86 % |
| 24,0 h | 0 % | 53 % | 3 % | 0 % | 97 % | 84 % | 67 % |

Zur Bestimmung der Alkoholdehydrogenaseaktivität in reinem Schweine-Magensaft ohne Futterbrei mit einem pH-Wert von etwa 3, in Schweine-Darminhalt mit einem pH-Wert von etwa 6 sowie in Schweine-Speichel und Menschen-Speichel, wurden 300 nM aktivierte Alkoholdehydrogenase der SEQ ID-Nr. 1, etwa 20 ppm DON mit jeweils 1 ml Magensaft (steril filtriert), 1 ml breiigem Darminhalt bzw. 1 ml Speichel gemischt. Als negative Kontrolle wurden Ansätze mitgeführt, die nur Verdauungsflüssigkeiten mit 20 ppm DON enthielten, und als positive Kontrolle dienten Transformationsansätze, die alle Komponenten inklusive 20 mM des synthetischen Redoxcofaktors PFC(III) enthielten. Die Probennahme erfolgte zu den Zeitpunkten 0; 3,0; 5,0 und 24,0 Stunden, wobei je Zeitpunkt 0,1 ml Probe entnommen wurden und die Reaktion mit 0,1 ml Methanol gestoppt wurde. Die Proben wurden bei -20 °C gelagert, aufgetaut und für 10 min bei 13.000 Upm mit einer Eppendorf Tischzentrifuge zentrifugiert und steril filtriert (0,2 µM Spartan Filter). Für die LC-MS/MS wurden die Proben im Laufmittel (siehe Beispiel 3) 1:10 verdünnt und so wie in Beispiel 3 mittels LC-MS/MS analysiert. Tabelle 9 zeigt die jeweiligen DON Konzentrationen, die zum Zeitpunkt der Probenentnahme gemessen wurden. Überraschenderweise erfolgte eine Reduktion von DON im Speichel ohne extern zugegebenem synthetischem Redoxcofaktor (unabhängig von der Spezies). Dies zeigt klar, dass in den Speichelsekreten von Mensch und Schwein Substanzen vorliegen, die als natürliche Redoxcofaktoren für die Transformation von DON mit der Alkoholdehydrogenase der SEQ ID-Nr. 1 geeignet sind. Im reinen Magensaft ohne Futterbrei wurde keine wesentliche Reduktion der DON Konzentration gemessen. Im Darminhalt erfolgt nur durch Zusatz des synthetischen Redoxcofaktors eine Abnahme der DON Konzentration.

**Tabelle 9:**

| | | DON [ppm] | | | |
|---|---|---|---|---|---|
| Probe | | 0 h | 3 h | 5 h | 24 h |
| Speichel (Mensch) | negative Kontrolle | 20 | 19 | 18 | 18 |
| | 0 mM PFC (III) | 20 | 13 | 12 | 8 |
| | positive Kontrolle 20 mM PFC (III) | 18 | 0 | 0 | 0 |
| Speichel (Schwein) | negative Kontrolle | 20 | 20 | 19 | 18 |
| | 0 mM PFC(III) | 21 | 10 | 8 | 5 |
| | positive Kontrolle 20 mM PFC (III) | 20 | 0 | 0 | 0 |
| Magensaft | negative Kontrolle | 22 | 22 | 21 | 21 |
| | 0 mM PFC(III) | 22 | 21 | 21 | 20 |
| | positive Kontrolle 20 mM PFC (III) | 24 | 21 | 19 | 18 |
| Darminhalt | negative Kontrolle | 21 | 20 | 20 | 20 |
| | 0 mM PFC(III) | 24 | 23 | 22 | 22 |
| | positive Kontrolle 20 mM PFC (III) | 23 | 9 | 8 | 4 |

Zur Bestimmung der Aktivität der Alkoholdehydrogenasen in Ferkelaufzuchtfutter (FAF) wurden je 100 mg FAF vermischt mit 400 µl 100 mM Tris-HCl Puffer, pH 7,5, 400 µl Speichel vom Schwein, 400 µl sterilem Magensaft vom Schwein oder 400 µl Darminhalt vom Schwein herangezogen. Diese FAF Suspensionen wurden über Nacht bei 4 °C gelagert. Danach erfolgte die Zugabe von etwa 20 ppm DON, und/oder 300 nM aktivierter Alkoholdehydrogenase der SEQ ID-Nr. 1, und/oder 20 mM des synthetischen Redoxcofaktors PFC(III) zu allen Proben. Als negative Kontrolle dienten die Ansätze ohne Alkoholdehydrogenase und ohne den externen synthetischen Redoxcofaktor. Als positive Kontrolle dienten die Ansätze mit Zugabe der Alkoholdehydrogenase und dem synthetischen Redoxcofaktor. Die Probennahme erfolgte zu den Zeitpunkten 0; 3,0; 5,0 und 24,0 Stunden. Pro Zeitpunkt wurde eine Probe zur Gänze verwendet. Zur Probe wurden 500 µl Methanol zugegeben, gefolgt von 30 min Homogenisierung auf einem Schüttler mit 300 Upm. Danach wurden die Proben für 15 min zentrifugiert (Eppendorf Tischzentrifuge 13.000 Upm) und der Überstand mit einer Spritze durch ein 0,2 µM Spartan Filter filtriert. Die Überstände wurden bei -20 °C gelagert, aufgetaut und für die LC-MS/MS im Laufmittel 1:10 verdünnt, und so wie in Beispiel 3 mittels LC-MS/MS analysiert.

Tabelle 10 zeigt die DON Konzentration, die zu den jeweiligen Zeitpunkten in den Proben vorlag. In der FAF-Puffermischung befinden sich Substanzen, die die Rolle der extern zugegebenen synthetischen Redoxcofaktoren übernehmen können, da die DON Konzentration in Abwesenheit des externen synthetischen Redoxcofaktors kontinuierlich abnimmt. Diese Substanzen stammen aus dem Ferkelaufzuchtfutter, da wie zuvor gezeigt, keine DON Transformation in Puffer ohne externen synthetischen Redoxcofaktor gemessen werden konnte. In Anwesenheit des externen synthetischen Redoxcofaktors verläuft die Transformation von DON in der FAF-Puffermischung im Vergleich schneller.

In der Mischung aus FAF und Speichel zeigte die Alkoholdehydrogenase ebenfalls Aktivität unabhängig vom Vorhandensein des externen synthetischen Redoxcofaktors. Wobei in den Transformationsansätzen, die den externen synthetischen Redoxcofaktor enthielten eine schnellere Reduktion von DON erfolgte.

Überraschenderweise ist die Alkoholdehydrogenase der SEQ ID-Nr. 1 in der FAF-Magensaftmischung ohne Zugabe des externen synthetischen Redoxcofaktors ebenfalls aktiv. Durch die Zugabe von FAF zum Magensaft wurde einerseits der pH des Magensaftes erhöht und andererseits natürlich vorkommende Redoxcofaktoren aus dem FAF gelöst, die den externen synthetischen Redoxcofaktor ersetzen können. Im Darminhalt wurde nur dann Aktivität der Alkoholdehydrogenase festgestellt, wenn ein externer synthetischer Redoxcofaktor zum Transformationsansatz zugegeben wurde.

**Tabelle 10:**

| | | DON [ppm] | | | |
|---|---|---|---|---|---|
| Probe | | 0 h | 3 h | 5 h | 24 h |
| FAF in Puffer | negative Kontrolle | 21 | 20 | 20 | 20 |
| | 0 mM PFC(III) | 20 | 10 | 9 | 5 |
| | positive Kontrolle 20 mM PFC(III) | 21 | 0 | 0 | 0 |
| FAF in Speichel | negative Kontrolle | 20 | 20 | 20 | 20 |
| | 0 mM PFC(III) | 20 | 12 | 9 | 8 |
| | positive Kontrolle 20 mM PFC (III) | 21 | 1 | 0,8 | 0,5 |
| FAF in Magensaft | negative Kontrolle | 21 | 21 | 20 | 20 |
| | 0 mM PFC(III) | 20 | 7 | 5 | 2 |
| | positive Kontrolle 20 mM PFC(III) | 20 | 5 | 0,7 | 0 |
| FAF in Darminhalt | negative Kontrolle | 21 | 20 | 20 | 20 |
| | 0 mM PFC(III) | 21 | 20 | 18 | 16 |
| | positive Kontrolle 20 mM PFC(III) | 20 | 5 | 3 | 0,7 |

## Patentansprüche

1. Verwendung von einer Metallionen und einen Chinoncofaktor enthaltenden Alkoholdehydrogenase der Sequenz ID-Nr. 1 oder dazu eine Sequenzidentität von wenigstens 80 %, bevorzugt wenigstens 86 %, insbesondere bevorzugt wenigstens 89 % aufweisenden funktionellen Variante und wenigstens einem Redoxcofaktor zur Transformation von wenigstens einem am C-3 Atom eine Hydroxygruppe aufweisenden Trichothecen.

2. Verwendung nach Anspruch 1, **dadurch gekennzeichnet, dass** die Aminosäuresequenz der funktionellen Variante aus der Gruppe der Sequenz ID-Nrn. 2 bis 4 gewählt wird.

3. Verwendung nach Anspruch 1 oder 2, **dadurch gekennzeichnet, dass** der Chinoncofaktor aus der Gruppe PCC, TTC, TPC, LTC und CTC, vorzugsweise PCC gewählt wird.

4. Verwendung nach Anspruch 1, 2 oder 3, **dadurch gekennzeichnet, dass** der Chinoncofaktor mittels wenigstens einem Metallion gewählt aus der Gruppe Li⁺, Na⁺, K⁺, Mg²⁺, Ca²⁺, Zn²⁺, Zn³⁺, Mn²⁺, Mn³⁺, Fe²⁺,Fe³⁺, Cu²⁺, Cu³⁺, Co²⁺ und Co³, vorzugsweise Ca²⁺ und Mg²⁺ an die Alkoholdehydrogenase gebunden wird.

5. Verwendung nach einem der Ansprüche 1 bis 4, **dadurch gekennzeichnet, dass** der wenigstens eine Redoxcofaktor aus der Gruppe PMS, PMS-Derivate, Kaliumhexacyanidoferrat (III), Natriumhexacyanidoferrat (III), Cytochrome C, Coenzym Q1, Coenzym Q10, Methylenblau und TMPD, vorzugsweise PMS, Coenzym Q1 und Coenzym Q10 gewählt wird.

6. Verwendung nach einem der Ansprüche 1 bis 5, **dadurch gekennzeichnet, dass** die Transformation von am C-3 Atom eine Hydroxygruppe aufweisenden Trichothecenen in Nahrungs- oder Futtermitteln, insbesondere für Schweine, Geflügel, Rinder, Pferde, Fische, Aquakultur und Haustiere sowie in für die zur Herstellung oder Verarbeitung von Nahrungs- oder Futtermitteln eingesetzten pflanzlichen Rohstoffen durchgeführt wird.

7. Verfahren zur enzymatischen Transformation von Trichothecenen, **dadurch gekennzeichnet, dass** wenigstens ein am C-3 Atom eine Hydroxygruppe aufweisendes Trichothecen mit einer Metallionen und einen Chinoncofaktor enthaltenden Alkoholdehydrogenase der Sequenz ID-Nr. 1 oder mit einer dazu eine Sequenzidentität von wenigstens 80 %, bevorzugt von wenigstens 86 %, insbesondere bevorzugt von wenigstens 89 % aufweisenden funktionellen Variante, wobei die enzymatische Funktion der funktionellen Variante im Wesentlichen beibehalten wird, mit wenigstens einem Redoxcofaktor sowie Wasser und gegebenenfalls zusätzlich mit wenigstens einem Hilfsstoff in Kontakt gebracht wird.

8. Verfahren nach Anspruch 7, **dadurch gekennzeichnet, dass** die Aminosäuresequenz der funktionellen Variante aus der Gruppe der Sequenz ID-Nrn. 2 bis 4 gewählt wird.

9. Verfahren einem der Ansprüche 7 oder 8, **dadurch gekennzeichnet, dass** das am C-3 Atom eine Hydroxygruppe aufweisende Trichothecen bei einer Temperatur zwischen 5 °C und 55 °C, bevorzugt zwischen 10 °C und 50 °C, insbesondere bevorzugt zwischen 28 °C und 45 °C transformiert wird.

10. Verfahren nach einem der Ansprüche 7, 8 oder 9, **dadurch gekennzeichnet, dass** das wenigstens eine am C-3 Atom eine Hydroxygruppe aufweisende Trichothecen für wenigstens eine Minute, bevorzugt für wenigstens 5 Minuten, insbesondere bevorzugt für wenigstens 60 Minuten mit der Metallionen und einen Chinoncofaktor enthaltenden Alkoholdehydrogenase oder wenigstens einer funktionellen Variante davon, dem Redoxcofaktor, Wasser sowie gegebenenfalls dem Hilfsstoff in Kontakt gebracht wird.

11. Verfahren nach einem der Ansprüche 7 bis 10, **dadurch gekennzeichnet, dass** der Chinoncofaktor aus der Gruppe PCC, TTC, TPC, LTC und CTC, vorzugsweise PCC gewählt wird.

12. Verfahren nach einem der Ansprüche 7 bis 11, **dadurch gekennzeichnet, dass** der Chinoncofaktor mittels wenigstens einem Metallionen gewählt aus der Gruppe Li⁺, Na⁺, K⁺, Mg²⁺, Ca²⁺, Zn²⁺, Zn³⁺, Mn²⁺, Mn³⁺, Fe²⁺,Fe³⁺, Cu²⁺, Cu³⁺, Co²⁺ und Co³⁺, vorzugsweise Ca²⁺ und Mg²⁺ an die Alkoholdehydrogenase gebunden wird.

13. Verfahren nach einem der Ansprüche 7 bis 12, **dadurch gekennzeichnet, dass** der wenigstens eine Redoxcofaktor aus der Gruppe PMS, PMS-Derivate, Kaliumhexacyanidoferrat (III), Natriumhexacyanidoferrat (III), Cytochrome C, Coenzym Q1, Coenzym Q10, Methylenblau, TMPD, vorzugsweise PMS, Coenzym Q1 und Coenzym Q10 gewählt wird.

14. Trichothecene transformierender Zusatzstoff, **dadurch gekennzeichnet, dass** der Zusatzstoff eine Metallionen und einen Chinoncofaktor enthaltende Alkoholdehydrogenase der Sequenz ID-Nr. 1 oder eine dazu eine Sequenzidentität von wenigstens 80 %, bevorzugt von wenigstens 86 %, insbesondere bevorzugt von wenigstens 89 % aufweisende funktionelle Variante und gegebenenfalls zusätzlich wenigstens eine weitere Komponente gewählt aus der Gruppe bestehend aus einem Redoxcofaktor und wenigstens einem Hilfsstoff enthält.

15. Zusatzstoff nach Anspruch 14, **dadurch gekennzeichnet, dass** er eine funktionelle Variante der Alkoholdehydrogenase der Sequenz ID-Nr. 1 gewählt aus der Gruppe der Sequenz ID-Nrn. 2 bis 4 enthält.

16. Zusatzstoff nach Anspruch 14 oder 15, **dadurch gekennzeichnet, dass** er einen Chinoncofaktor gewählt aus der Gruppe PCC, TTC, TPC, LTC und CTC, vorzugsweise PCC enthält.

17. Zusatzstoff nach Anspruch 14, 15 oder 16, **dadurch gekennzeichnet, dass** er den durch wenigstens ein Metallion gewählt aus der Gruppe Li⁺, Na⁺, K⁺, Mg²⁺, Ca²⁺, Zn²⁺, Zn³⁺, Mn²⁺, Mn³⁺, Fe²⁺,Fe³⁺, Cu²⁺, Cu³⁺, Co²⁺ und Co³⁺, vorzugsweise Ca²⁺ und Mg²⁺ an die Alkoholdehydrogenase gebundenen Chinoncofaktor enthält.

18. Zusatzstoff nach einem der Ansprüche 14 bis 17, **dadurch gekennzeichnet, dass** als Redoxcofaktor ein synthetischer Redoxcofaktor gewählt ist aus Gruppe PMS, PMS-Derivate, Kaliumhexacyanidoferrat (III), Natriumhexacyanidoferrat (III), Cytochrome C, Coenzym Q1, Coenzym Q10, Methylenblau und TMPD, vorzugsweise PMS, Coenzym Q1 und Coenzym Q10 enthalten ist.

19. Zusatzstoff nach einem der Ansprüche 14 bis 18 **dadurch gekennzeichnet, dass** der Hilfsstoff gewählt aus Gruppe: inerte Träger, Vitamine, Mineralstoffe, phytogene Substanzen, Enzyme und weitere Komponenten zur Detoxifizierung von Mykotoxinen, wie Mykotoxin-abbauende Enzyme, insbesondere Aflatoxin-Oxidasen, Ergotamin-Hydrolasen, Ergotamin-Amidasen, Zearalenon-Esterasen, Zearalenon-Lactonasen, Zearalenon-Hydrolasen, Ochratoxin-Amidasen, Fumonisin-Aminotransferasen, Fumonisin-Carboxyltransferasen, Aminopolyol-Aminoxidasen, Deoxynivalenol-Epoxidhydrolasen, Deoxynivalenol- Dehydrogenasen, Deoxynivalenol-Oxidasen, Trichothecene-Dehydrogenasen, Trichothecene-Oxidasen; und Mykotoxintransformierende Mikroorganismen wie z.B. DSM 11798; und Mykotoxin-bindende Stoffe, beispielsweise mikrobielle Zellwände oder anorganische Materialien, wie Bentonite, enthalten ist.

20. Zusatzstoff nach einem der Ansprüche 14 bis 19, **dadurch gekennzeichnet, dass** er in verkapselter oder gecoateter Form vorliegt.

21. Verwendung von wenigstens einem Zusatzstoff nach einem der Ansprüche 14 bis 20 zur Herstellung eines Präparats zur Prophylaxe und/oder Behandlung von Trichothecen-Mykotoxikosen, bevorzugt von durch am C-3 Atom eine Hydroxygruppe aufweisenden Trichothecenen versursachten Mykotoxikosen, insbesondere von Deoxynivalenol-Mykotoxikosen.

## Claims

1. Use of an alcohol dehydrogenase of SEQ ID no. 1 containing metal ions and a quinone cofactor, or in addition, a functional variant exhibiting a sequence identity of at least 80%, preferably 86%, especially preferred at least 89%, and at least one redox cofactor for the transformation of at least one trichothecene exhibiting a hydroxyl group on the C-3 atom.

2. Use according to Claim 1 **characterised in that** the amino acid sequence of the functional variant is selected from the group of sequence ID numbers 2 to 4.

3. Use according to Claim 1 or 2 **characterised in that** the quinone cofactor is selected from the group PCC, TTC, TPC, LTC, and CTC, preferably PCC.

4. Use according to Claim 1, 2, or 3 **characterised in that** the quinone cofactor is bound to the alcohol dehydrogenase by at least one metal ion selected from the group Li⁺, Na⁺, K⁺, Mg²⁺, Ca²⁺, Zn²⁺, Zn³⁺, Mn²⁺, Mn³⁺, Fe²⁺, Fe³⁺, Cu²⁺, Cu³⁺, Co²⁺ and Co³, preferably Ca²⁺ and Mg²⁺.

5. Use according to one of the Claims 1 to 4 **characterised in that** at least one redox cofactor is selected from the group PMS, PMS derivatives, potassium hexacyanoferrate (III), sodium hexacyanoferrate (III), cytochrome C, coenzyme Q1, coenzyme Q10, methylene blue, and TMPD, preferably PMS, coenzyme Q1 and coenzyme Q10.

6. Use according to one of the Claims 1 to 5 **characterised in that** the transformation of trichothecenes exhibiting a hydroxyl group on the C-3 atom in food and feed, especially for swine, poultry, cattle, horses, fish, aquaculture, and domestic animals, and in plant-based raw materials used for the production or processing of food and feed, is carried out.

7. Procedure for the enzymatic transformation of trichothecenes, **characterised in that** at least one trichothecene exhibiting a hydroxyl group on the C-3 atom is brought into contact with an alcohol dehydrogenase of sequence ID no. 1 containing metal ions and a quinone cofactor, or with a functional variant additionally exhibiting a sequence identity of at least 80%, preferably at least 86%, especially preferred at least 89% with at least one redox cofactor and water, and if necessary at least one excipient.

8. Procedure according to Claim 7 **characterised in that** the amino acid sequence of the functional variant is selected from the group of sequence ID numbers 2 to 4.

9. Procedure according to one of the Claims 7 or 8 **characterised in that** the trichothecene exhibiting a hydroxyl group on the C-3 atom is transformed at a temperature between 5°C and 55°C, preferably between 10°C and 50°C, especially preferred between 28°C and 45°C.

10. Procedure according to one of the Claims 7, 8, or 9 **characterised in that** at least one trichothecene exhibiting a hydroxyl group on the C-3 atom is brought into contact with the alcohol dehydrogenase containing metal ions and a quinone cofactor, or at least a functional variant thereof, with the redox factor, with water, and if necessary, with the excipient, for at least one minute, preferably at least 5 minutes, especially at least 60 minutes.

11. Procedure according to one of the Claims 7 to 10 **characterised in that** the quinone cofactor is selected from the group PCC, TTC, TPC, LTC, and CTC, preferably PCC.

12. Procedure according to one of the Claims 7 to 11 **characterised in that** the quinone cofactor is bound to the alcohol dehydrogenase by at least one metal ion selected from the group Li⁺, Na⁺, K⁺, Mg²⁺, Ca²⁺, Zn²⁺, Zn³⁺, Mn²⁺, Mn³⁺, Fe²⁺, Fe³⁺, Cu²⁺, Cu³⁺, Co²⁺ and Co³⁺, preferably Ca²⁺ and Mg²⁺.

13. Procedure according to one of the Claims 7 to 12 **characterised in that** at least one redox cofactor is selected from the group PMS, PMS derivatives, potassium hexacyanoferrate (III), sodium hexacyanoferrate (III), cytochrome C, coenzyme Q1, coenzyme Q10, methylene blue, TMPD, preferably PMS, coenzyme Q1, and coenzyme Q10.

14. Trichothecene-transforming additive **characterised in that** the additive contains an alcohol dehydrogenase of sequence ID no. 1 containing metal ions and a quinone cofactor, or a functional variant additionally exhibiting a sequence identity of at least 80%, preferably at least 86%, especially preferred at least 89%, and if necessary, additionally at least one additional component selected from the group consisting of a redox cofactor and at least one excipient.

15. Additive according to Claim 14 **characterised in that** it contains a functional variant of the alcohol dehydrogenase of sequence ID no. 1 selected from the group of sequence ID numbers 2 to 4.

16. Additive according to Claim 14 or 15 **characterised in that** it contains a quinone cofactor selected from the group PCC, TTC, TPC, LTC, and CTC, preferably PCC.

17. Additive according to Claim 14, 15, or 16 **characterised in that** it contains the quinone cofactor bound to the alcohol dehydrogenase by at least one metal ion selected from the group Li⁺, Na⁺, K⁺, Mg²⁺, Ca²⁺, Zn²⁺, Zn³⁺, Mn²⁺, Mn³⁺, Fe²⁺, Fe³⁺, Cu²⁺, Cu³⁺, Co²⁺ and Co³⁺, preferably Ca²⁺ and Mg²⁺.

18. Additive according to one of the Claims 14 to 17 **characterised in that** a synthetic redox cofactor is selected from the group PMS, PMS derivatives, potassium hexacyanoferrate (III), sodium hexacyanoferrate (III), cytochrome C, coenzyme Q1, coenzyme Q10, methylene blue, and TMPD, preferably PMS, coenzyme Q1, and coenzyme Q10 as the redox cofactor.

19. Additive according to one of the Claims 14 to 18 **characterised in that** the excipient is selected from a group of inert carriers, vitamins, mineral substances, phytogenetic substances, enzymes, and additional components for the detoxification of mycotoxins like mycotoxin-degrading enzymes, especially aflatoxin-oxidases, ergotamine hydrolases, ergotamine amidases, zearalenone esterases, zearalenone lactonases, zearalenone hydrolases, ochratoxin amidases, fumonisin aminotransferases, fumonisin carboxyltransferases, amino polyol amine oxidases, deoxynivalenol epoxide hydrolases, deoxynivalenol dehydrogenases, deoxynivalenol oxidases, trichothecene dehydrogenases, trichothecene oxidases; and mycotoxin-transforming microorganisms such as DSM 11798; and mycotoxin-binding substances such as microbial cell walls or inorganic materials like bentonite.

20. Additive according to one of the Claims 14 to 19 **characterised in that** it is present in an encapsulated or coated form.

21. Use of at least one additive according to one of the Claims 14 to 20 for the production of a compound for the prevention and/or treatment of trichothecene mycotoxicoses, preferably of mycotoxicoses caused by trichothecenes that exhibit a hydroxyl group on the C-3 atom, especially such as deoxynivalenol mycotoxicoses.

## Revendications

1. Utilisation d'un alcool déshydrogénase de la séquence ID no 1 contenant des ions métalliques et un cofacteur quinonique ou, en plus, d'une variante fonctionnelle présentant une identité de séquence d'au moins 80 %, de manière préférée d'au moins 86 %, de manière particulièrement préférée d'au moins 89 % et d'au moins un cofacteur redox pour la transformation d'au moins un trichothécène présentant un groupe hydroxy sur l'atome C-3.

2. Utilisation selon la revendication 1, **caractérisée en ce que** la séquence d'acides aminés de la variante fonctionnelle est choisie parmi le groupe de séquences ID no 2 à 4.

3. Utilisation selon la revendication 1 ou 2, **caractérisée en ce que** le cofacteur quinonique est choisi parmi le groupe PCC, TTC, TPC, LTC et CTC, de préférence PCC.

4. Utilisation selon la revendication 1, 2 ou 3, **caractérisée en ce que** le cofacteur quinonique est lié à l'alcool déshydrogénase au moyen d'au moins un ion métallique choisi parmi le groupe Li⁺, Na⁺, K⁺, Mg²⁺, Ca²⁺, Zn²⁺, Zn³⁺, Mn²⁺, Mn³⁺, Fe²⁺, Fe³⁺, Cu²⁺, Cu³⁺, Co²⁺ et Co³⁺, de préférence Ca²⁺ et Mg²⁺.

5. Utilisation selon l'une des revendications 1 à 4, **caractérisée en ce que** l'au moins un cofacteur redox est choisi parmi le groupe PMS, dérivés PMS, ferrate d'hexacyanure de potassium (III), hexacyanidoferrate de sodium (III), cytochrome C, coenzyme Q1, coenzyme Q10, bleu de méthylène et TMPD, de préférence PMS, coenzyme Q1 et coenzyme Q10.

6. Utilisation selon l'une des revendications 1 à 5, **caractérisée en ce que** la transformation de trichothécènes présentant sur l'atome C-3 un groupe hydroxy est mise en œuvre dans des denrées alimentaires ou aliments pour animaux, en particulier pour porcs, volailles, bovins, chevaux, poissons, l'aquaculture et les animaux domestiques, ainsi que dans les matières premières végétales utilisées pour la fabrication ou la transformation de denrées alimentaires ou d'aliments pour animaux.

7. Procédé de transformation enzymatique de trichothécènes, **caractérisé en ce qu'**au moins un trichothécène présentant sur l'atome C-3 un groupe hydroxy est mis en contact avec un alcool déshydrogénase de la séquence ID no 1 contenant des ions métalliques et un cofacteur quinonique ou avec une variante fonctionnelle présentant à cet effet une identité de séquence d'au moins 80 %, de manière préférée d'au moins 86 %, en particulier de manière préférée d'au moins 89 %, dans lequel la fonction enzymatique de la variante fonctionnelle est sensiblement maintenue, avec au moins un cofacteur redox ainsi qu'avec de l'eau et éventuellement en plus avec au moins un excipient.

8. Procédé selon la revendication 7, **caractérisé en ce que** la séquence d'acides aminés de la variante fonctionnelle est choisie parmi le groupe de séquences ID no 2 à 4.

9. Procédé selon l'une des revendications 7 ou 8, **caractérisé en ce que** le trichothécène présentant un groupe hydroxy sur l'atome C-3 est transformé à une température entre 5 °C et 55 °C, de manière préférée entre 10 °C et 50 °C, de manière particulièrement préférée entre 28 °C et 45 °C.

10. Procédé selon l'une des revendications 7, 8 ou 9, **caractérisé en ce que** l'au moins un trichothécène présentant un groupe hydroxy sur l'atome C-3 est amené en contact pendant au moins une minute, de manière préférée pendant au moins 5 minutes, en particulier de manière préférée pendant au moins 60 minutes avec de l'alcool déshydrogénase contenant des ions métalliques et un cofacteur quinonique ou au moins une variante fonctionnelle de celui-ci, le cofacteur redox, de l'eau ainsi que, le cas échéant, l'excipient.

11. Procédé selon l'une des revendications 7 à 10, **caractérisé en ce que** le cofacteur quinonique est choisi parmi le groupe PCC, TTC, TPC, LTC et CTC, de préférence PCC.

12. Procédé selon l'une des revendications 7 à 11, **caractérisé en ce que** le cofacteur quinonique est lié à l'alcool déshydrogénase au moyen d'au moins un ion métallique choisi parmi le groupe Li⁺, Na⁺, K⁺, Mg²⁺, Ca²⁺, Zn²⁺, Zn³⁺, Mn²⁺, Mn³⁺, Fe²⁺, Fe³⁺, Cu²⁺, Cu³⁺, Co²⁺ et Co³⁺, de préférence Ca²⁺ et Mg²⁺.

13. Procédé selon l'une des revendications 7 à 12, **caractérisé en ce que** l'au moins un cofacteur redox est choisi parmi le groupe PMS, dérivés PMS, fhexacyanidoferrate de potassium (III), hexacyanidoferrate de sodium (III), cytochrome C, coenzyme Q1, coenzyme Q10, bleu de méthylène, TMPD, de préférence PMS, coenzyme Q1 et coenzyme Q10.

14. Additif transformant du trichothécène, **caractérisé en ce que** l'additif contient un alcool déshydrogénase de la séquence ID no 1 contenant des ions métalliques et un cofacteur quinonique ou une variante fonctionnelle présentant à cet effet une identité de séquence d'au moins 80 %, de manière préférée d'au moins 86 %, en particulier de manière préférée d'au moins 89 % et éventuellement en plus au moins un autre composant choisi parmi le groupe constitué d'un cofacteur redox et d'au moins un excipient.

15. Additif selon la revendication 14, **caractérisé en ce qu'**il contient une variante fonctionnelle de l'alcool déshydrogénase de la séquence ID no 1 choisie parmi le groupe de séquences ID no 2 à 4.

16. Additif selon la revendication 14 ou 15, **caractérisé en ce qu'**il contient un cofacteur quinonique choisi parmi le groupe PCC, TTC, TPC, LTC et CTC, de préférence PCC.

17. Additif selon la revendication 14, 15 ou 16, **caractérisé en ce qu'**il contient le cofacteur quinonique lié à l'alcool déshydrogénase par au moins un ion métallique choisi parmi le groupe Li⁺, Na⁺, K⁺, Mg²⁺, Ca²⁺, Zn²⁺, Zn³⁺, Mn²⁺, Mn³⁺, Fe²⁺, Fe³⁺, Cu²⁺, Cu³⁺, Co²⁺ et Co³⁺, de préférence Ca²⁺ et Mg⁺.

18. Additif selon l'une des revendications 14 à 17, **caractérisé en ce qu'**est contenu en tant que cofacteur redox un cofacteur redox synthétique choisi parmi le groupe PMS, dérivés PMS, hexacyanidoferrate de potassium (III), hexacyanidoferrate de sodium (III), cytochrome C, coenzyme Q1, coenzyme Q10, bleu de méthylène et TMPD, de préférence PMS, coenzyme Q1 et coenzyme Q10.

19. Additif selon l'une des revendications 14 à 18, **caractérisé en ce qu'**est contenu l'additif choisi parmi le groupe : supports inertes, vitamines, minéraux, substances phytogènes, Enzymes et autres composants détoxifiants de mycotoxines, comme des enzymes dégradantes de mycotoxine, en particulier des oxydases d'aflatoxine, des hydrolases d'ergotamine, des amidases d'ergotamine, des estérases d'zéaralénone, des lactonases de zéaralénone, des hydrolases de zéaralénone, des amidases d'ochratoxine, des aminotransférases de fumonisine, des carboxyltransférases de fumonisine, des aminooxydases d'aminopolyol, des époxyhydrolases de déoxynivalénol, des déhydrogénases de déoxynivalénol, des oxydases de déoxynivalénol, des déshydrogénases de trichothécène, des oxydases de trichothécène ; et des micro-organismes transformateurs de mycotoxines tels que le DSM 11798 ; et des substances liant les mycotoxines, par exemple des parois cellulaires microbiennes ou des matériaux inorganiques comme la bentonite.

20. Additif selon l'une des revendications 14 à 19, **caractérisé en ce qu'**il est présent sous forme encapsulée ou revêtue.

21. Utilisation d'au moins un additif selon l'une des revendications 14 à 20 pour la fabrication d'une préparation pour la prophylaxie et/ou le traitement de mycotoxicoses aux trichothécènes, de manière préférée de mycotoxicoses provoquées par des trichothécènes présentant un groupe hydroxy sur l'atome C-3, en particulier de mycotoxicoses au désoxynivalénol.
